(19) Europäisches Patentamt
European Patent Office
Office européen des brevets

(11) **EP 4 567 040 A1**

(12) **EUROPEAN PATENT APPLICATION**
published in accordance with Art. 153(4) EPC


(43) Date of publication:
**11.06.2025 Bulletin 2025/24**

(21) Application number: **23849339.9**

(22) Date of filing: **31.07.2023**

(51) International Patent Classification (IPC):
***C07D 498/06*** *(2006.01)* ***A61K 31/5383*** *(2006.01)*
***A61P 35/00*** *(2006.01)*

(52) Cooperative Patent Classification (CPC):
**A61K 31/5383; A61P 35/00; C07D 498/06**

(86) International application number:
**PCT/CN2023/110180**

(87) International publication number:
**WO 2024/027631 (08.02.2024 Gazette 2024/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **01.08.2022 CN 202210915848**



(71) Applicant: **Jiangsu Hengrui Pharmaceuticals Co., Ltd.**
**Lianyungang, Jiangsu 222047 (CN)**

(72) Inventors:
- **SU, Xiaoming**
  **Lianyungang, Jiangsu 222047 (CN)**
- **LI, Zhiya**
  **Lianyungang, Jiangsu 222047 (CN)**
- **HU, Yimin**
  **Lianyungang, Jiangsu 222047 (CN)**
- **XU, Gujun**
  **Lianyungang, Jiangsu 222047 (CN)**
- **SHANG, Tingting**
  **Lianyungang, Jiangsu 222047 (CN)**
- **WANG, Jie**
  **Lianyungang, Jiangsu 222047 (CN)**

(74) Representative: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**


# (54) CRYSTAL FORM OF FUSED TRICYCLIC DERIVATIVE AND PREPARATION METHOD


(57) The present disclosure relates to a crystal form of a fused tricyclic derivative and a preparation method, and specifically relates to a crystal form of a compound (3S,4R)-4-((5-chloro-4-((S)-8-fluoro-2-(2-hydroxypropane-2-yl)-3-methyl-3,4-dihydro-5-oxa-1,2a-diazacenaphthylene-6-yl)pyrimidine-2-yl)amino)tetrahydro-2H-pyran-3-ol, a pharmaceutically acceptable salt thereof, and a corresponding preparation method.


EP 4 567 040 A1

## Description

The present application claims priority to Chinese Patent Application No. 202210915848.1 filed on August 1, 2022, which is incorporated herein by reference in its entirety.

## TECHNICAL FIELD

The present disclosure relates to crystal forms of a fused tricyclic derivative and preparation methods therefor and pertains to the field of pharmaceuticals.

## BACKGROUND

Cyclin-dependent kinases (CDKs) are important cellular enzymes that play an important role in regulating eukaryotic cell division and proliferation. The cyclin-dependent kinase catalytic units are activated by regulatory subunits known as cyclins. At least 16 mammalian cyclins have been identified (Annu. Rev. Pharmacol. Toxicol. (1999) 39:295-312). Cyclin B/CDK1, cyclin A/CDK2, cyclin E/CDK2, cyclin D/CDK4, cyclin D/CDK6, and other possible heterodynes are important regulators of cell cycle progression. Additional functions of cyclin/CDK heterodynes include regulation of transcription, DNA repair, differentiation, and apoptosis (Annu. Rev. Cell. Dev. Biol. (1997) 13:261-291).

PCT/CN2022/074509 provides a class of new cyclin-dependent kinase inhibitors. The search for a development form for the compounds disclosed in the application has important clinical significance.

## SUMMARY

The present disclosure provides crystal forms of the compound (3S,4R)-4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)tetrahydro-2H-pyran-3-ol (the compound represented by formula (I)), pharmaceutically acceptable salts thereof, and corresponding preparation methods therefor,

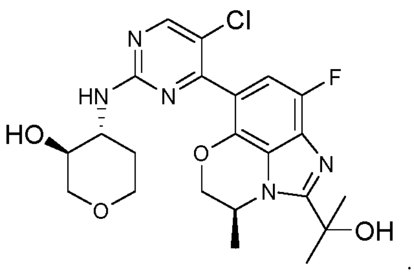

(I)

The present disclosure provides a crystal form A of the compound represented by formula (I), and an X-ray powder diffraction pattern, expressed in terms of 2θ angles, which are diffraction angles, of the crystal form has characteristic peaks at 9.6, 10.0, 11.7, 15.0, 21.1, and 21.7.

In an optional embodiment, the X-ray powder diffraction pattern, expressed in terms of 2θ angles, which are diffraction angles, of the crystal form A of the compound represented by formula (I) provided in the present disclosure has characteristic peaks at 7.8, 9.6, 10.0, 11.7, 15.0, 18.9, 19.4, 20.1, 21.1, and 21.7.

In an optional embodiment, the X-ray powder diffraction pattern, expressed in terms of 2θ angles, which are diffraction angles, of the crystal form A of the compound represented by formula (I) provided in the present disclosure has characteristic peaks at 7.8, 9.6, 10.0, 11.7, 15.0, 18.9, 19.4, 20.1, 21.1, 21.7, 23.5, 26.7, and 29.4.

In an optional embodiment, the X-ray powder diffraction pattern, expressed in terms of 2θ angles, which are diffraction angles, of the crystal form A of the compound represented by formula (I) provided in the present disclosure is shown in FIG. 2.

The present disclosure provides a preparation method for the crystal form A of the compound represented by formula (I), the method comprising the following steps:

method 1

a) dissolving the compound represented by formula (I) in a solvent 1, and
b) adding a solvent 2 and crystallizing,

wherein the solvent 1 is selected from the group consisting of a ketone solvent and an ester solvent, and the solvent 2 is an ether solvent.
In an optional embodiment, the ketone solvent is selected from the group consisting of acetone and methyl isobutyl ketone, the ester solvent is ethyl acetate, and the ether solvent is selected from the group consisting of isopropyl ether and methyl tert-butyl ether.

method 2
dissolving the compound represented by formula (I) in a solvent 3 and stirring for crystallization, wherein the solvent 3 is selected from the group consisting of an ester solvent and a ketone solvent; preferably, the ester solvent is selected from the group consisting of ethyl acetate and isopropyl acetate, and the ketone solvent is methyl isobutyl ketone.

The present disclosure provides a crystal form B of the compound represented by formula (I), and an X-ray powder diffraction pattern, expressed in terms of 2θ angles, which are diffraction angles, of the crystal form has characteristic peaks at 11.3, 15.1, 20.9, 22.8, and 23.7.
In an optional embodiment, the X-ray powder diffraction pattern, expressed in terms of 2θ angles, which are diffraction angles, of the crystal form B of the compound represented by formula (I) provided in the present disclosure has characteristic peaks at 7.4, 11.3, 14.3, 15.1, 16.3, 18.0, 20.0, 20.9, 22.8, 23.2, 23.7, 25.1, and 26.0.
In an optional embodiment, the X-ray powder diffraction pattern, expressed in terms of 2θ angles, which are diffraction angles, of the crystal form B of the compound represented by formula (I) provided in the present disclosure has characteristic peaks at 7.4, 9.5, 11.3, 12.4, 12.8, 13.7, 14.3, 15.1, 16.3, 18.0, 19.2, 20.0, 20.9, 22.8, 23.2, 23.7, 25.1, 26.0, 26.8, 28.2, 30.4, 32.7, 33.6, 34.3, 35.5, 38.4, 39.0, and 40.5.
In an optional embodiment, the X-ray powder diffraction pattern, expressed in terms of 2θ angles, which are diffraction angles, of the crystal form B of the compound represented by formula (I) provided in the present disclosure is shown in FIG. 3.
The present disclosure provides a preparation method for the crystal form B of the compound represented by formula (I), the method comprising the following steps: dissolving the compound represented by formula (I) in a substituted lower alkane and stirring for crystallization.
In an optional embodiment, the substituted lower alkane is nitromethane.
The present disclosure provides a crystal form C of the compound represented by formula (I), and an X-ray powder diffraction pattern, expressed in terms of 2θ angles, which are diffraction angles, of the crystal form has characteristic peaks at 4.8, 10.1, 12.0, and 15.0.
In an optional embodiment, the X-ray powder diffraction pattern, expressed in terms of 2θ angles, which are diffraction angles, of the crystal form C of the compound represented by formula (I) provided in the present disclosure has characteristic peaks at 4.8, 7.6, 10.1, 12.0, 15.0, 19.7, 21.2, and 23.5.
In an optional embodiment, the X-ray powder diffraction pattern, expressed in terms of 2θ angles, which are diffraction angles, of the crystal form C of the compound represented by formula (I) provided in the present disclosure is shown in FIG. 4.
The present disclosure provides a preparation method for the crystal form C of the compound represented by formula (I), and the method is selected from the group consisting of the following methods:

method 1

dissolving the compound represented by formula (I) in a solvent 4 and volatilizing for crystallization, wherein the solvent 4 is selected from the group consisting of a substituted lower alkane and a ketone solvent; optionally, the substituted lower alkane is selected from the group consisting of dichloromethane and 1,2-dichloroethane, and the ketone solvent is methyl isobutyl ketone;

method 2
dissolving the compound represented by formula (I) in a solvent 5 and stirring for crystallization, wherein the solvent 5 is selected from the group consisting of an aromatic hydrocarbon solvent; optionally, the aromatic hydrocarbon solvent is selected from the group consisting of p-xylene and toluene; and
method 3

a) dissolving the compound represented by formula (I) in a solvent 6, and
b) adding a solvent 7 and crystallizing,

wherein the solvent 6 is selected from the group consisting of an alcohol solvent, a ketone solvent, a nitrile solvent, and an ether solvent, and the solvent 7 is an ether solvent; optionally, the alcohol solvent is selected from the group consisting of isopropanol and ethanol, the ketone solvent is acetone, the nitrile solvent is acetonitrile, and the ether solvent is selected from the group consisting of tetrahydrofuran and isopropyl ether.

The present disclosure provides a crystal form D of the compound represented by formula (I), and an X-ray powder diffraction pattern, expressed in terms of 2θ angles, which are diffraction angles, of the crystal form has characteristic peaks at 10.0, 10.5, 17.0, 18.7, and 23.9.

In an optional embodiment, the X-ray powder diffraction pattern, expressed in terms of 2θ angles, which are diffraction angles, of the crystal form D of the compound represented by formula (I) provided in the present disclosure has characteristic peaks at 10.0, 10.5, 11.0, 13.4, 15.9, 17.0, 18.3, 18.7, 20.8, 23.9, and 28.0.

In an optional embodiment, the X-ray powder diffraction pattern, expressed in terms of 2θ angles, which are diffraction angles, of the crystal form D of the compound represented by formula (I) provided in the present disclosure has characteristic peaks at 10.0, 10.5, 11.0, 12.5, 13.4, 15.9, 17.0, 18.3, 18.7, 20.1, 20.8, 22.3, 23.9, 26.7, 28.0, 30.5, 31.0, 32.1, 33.1, 33.8, and 34.6.

In an optional embodiment, the X-ray powder diffraction pattern, expressed in terms of 2θ angles, which are diffraction angles, of the crystal form D of the compound represented by formula (I) provided in the present disclosure is shown in FIG. 5.

The present disclosure provides a preparation method for the crystal form D of the compound represented by formula (I), the method comprising the following steps: mixing the compound represented by formula (I) with a solvent 8 and stirring for crystallization, wherein the solvent 8 is an ether solvent or a mixed solvent of an alcohol solvent and an ether solvent; optionally, the alcohol solvent is methanol, ethanol, or isopropanol, and the ether solvent is isopropyl ether.

The present disclosure provides a crystal form E of the compound represented by formula (I), and an X-ray powder diffraction pattern, expressed in terms of 2θ angles, which are diffraction angles, of the crystal form has characteristic peaks at 4.8, 11.4, 14.4, 15.1, 16.4, 19.3, and 22.9.

In an optional embodiment, the X-ray powder diffraction pattern, expressed in terms of 2θ angles, which are diffraction angles, of the crystal form E of the compound represented by formula (I) provided in the present disclosure has characteristic peaks at 4.8, 7.4, 11.4, 14.4, 15.1, 16.4, 17.9, 19.3, 19.9, 20.9, 22.9, and 23.5.

In an optional embodiment, the X-ray powder diffraction pattern, expressed in terms of 2θ angles, which are diffraction angles, of the crystal form E of the compound represented by formula (I) provided in the present disclosure has characteristic peaks at 4.8, 7.4, 9.6, 11.4, 14.4, 15.1, 16.4, 17.9, 19.3, 19.9, 20.9, 22.9, 23.5, 24.2, 25.2, 25.9, 26.8, and 30.5.

In an optional embodiment, the X-ray powder diffraction pattern, expressed in terms of 2θ angles, which are diffraction angles, of the crystal form E of the compound represented by formula (I) provided in the present disclosure is shown in FIG. 6.

The present disclosure provides a preparation method for the crystal form E of the compound represented by formula (I), and the method is selected from the group consisting of the following methods:

method 1
dissolving the compound represented by formula (I) in a solvent 9 and stirring for crystallization, wherein the solvent 9 is a nitrile solvent; optionally, the nitrile solvent is acetonitrile; and
method 2

a) dissolving the compound represented by formula (I) in a solvent 10, and
b) adding a solvent 11 and crystallizing,

wherein the solvent 10 is selected from the group consisting of a ketone solvent, and the solvent 11 is an ether solvent; optionally, the ketone solvent is acetone, and the ether solvent is isopropyl ether.

The present disclosure provides a crystal form F of the compound represented by formula (I), and an X-ray powder diffraction pattern, expressed in terms of 2θ angles, which are diffraction angles, of the crystal form has characteristic peaks at 11.1, 11.4, 14.3, 15.1, 15.7, 19.2, and 22.0.

In an optional embodiment, the X-ray powder diffraction pattern, expressed in terms of 2θ angles, which are diffraction angles, of the crystal form F of the compound represented by formula (I) provided in the present disclosure has characteristic peaks at 9.1, 11.1, 11.4, 13.3, 14.3, 15.1, 15.7, 16.6, 17.9, 19.2, 20.0, 21.1, 22.0, 23.6, 24.6, and 26.1.

In an optional embodiment, the X-ray powder diffraction pattern, expressed in terms of 2θ angles, which are diffraction angles, of the crystal form F of the compound represented by formula (I) provided in the present disclosure has characteristic peaks at 4.8, 9.1, 9.8, 11.1, 11.4, 12.0, 13.3, 14.3, 15.1, 15.7, 16.6, 17.9, 19.2, 20.0, 21.1, 22.0, 22.5, 23.6, 24.2, 24.6, 25.2, 25.6, 26.1, 28.7, 30.3, and 35.0.

In an optional embodiment, the X-ray powder diffraction pattern, expressed in terms of 2θ angles, which are

diffraction angles, of the crystal form F of the compound represented by formula (I) provided in the present disclosure is shown in FIG. 7.

The present disclosure provides a preparation method for the crystal form F of the compound represented by formula (I), and the method is selected from the group consisting of the following methods:

method 1
dissolving the compound represented by formula (I) in a solvent 12 and stirring for crystallization, wherein the solvent 12 is a ketone solvent; optionally, the ketone solvent is methyl isobutyl ketone; and
method 2
dissolving the compound represented by formula (I) in tetrahydrofuran completely, adding isopropyl ether and a seed crystal of the crystal form F, and crystallizing.

The present disclosure provides a crystal form G of the compound represented by formula (I), and an X-ray powder diffraction pattern, expressed in terms of 2θ angles, which are diffraction angles, of the crystal form has characteristic peaks at 11.3, 15.3, 16.1, 17.9, 18.6, and 20.8.

In an optional embodiment, the X-ray powder diffraction pattern, expressed in terms of 2θ angles, which are diffraction angles, of the crystal form G of the compound represented by formula (I) provided in the present disclosure has characteristic peaks at 11.3, 15.3, 16.1, 17.9, 18.6, 20.8, 22.8, 23.1, 23.8, and 25.8.

In an optional embodiment, the X-ray powder diffraction pattern, expressed in terms of 2θ angles, which are diffraction angles, of the crystal form G of the compound represented by formula (I) provided in the present disclosure has characteristic peaks at 9.1, 11.3, 12.6, 13.8, 14.7, 15.3, 16.1, 17.0, 17.5, 17.9, 18.6, 19.6, 20.8, 21.6, 22.8, 23.1, 23.8, 24.3, 25.3, 25.8, 27.3, 27.8, 29.3, 30.8, 32.3, 33.6, 34.5, and 35.0.

In an optional embodiment, the X-ray powder diffraction pattern, expressed in terms of 2θ angles, which are diffraction angles, of the crystal form G of the compound represented by formula (I) provided in the present disclosure is shown in FIG. 8.

The present disclosure provides a preparation method for the crystal form G of the compound represented by formula (I), the method comprising the following steps: dissolving the compound represented by formula (I) in a mixed solvent of an alcohol solvent and an ether solvent and stirring for crystallization, wherein optionally, the alcohol solvent is selected from the group consisting of methanol, and the ether solvent is isopropyl ether.

The present disclosure provides a crystal form H of the compound represented by formula (I), and an X-ray powder diffraction pattern, expressed in terms of 2θ angles, which are diffraction angles, of the crystal form has characteristic peaks at 11.7, 12.3, 15.5, 19.4, and 22.3.

In an optional embodiment, the X-ray powder diffraction pattern, expressed in terms of 2θ angles, which are diffraction angles, of the crystal form H of the compound represented by formula (I) provided in the present disclosure has characteristic peaks at 11.7, 12.3, 15.5, 18.2, 19.4, 21.3, 22.3, 24.9, 27.1, and 28.1.

In an optional embodiment, the X-ray powder diffraction pattern, expressed in terms of 2θ angles, which are diffraction angles, of the crystal form H of the compound represented by formula (I) provided in the present disclosure has characteristic peaks at 10.1, 11.7, 12.3, 12.8, 13.4, 15.5, 17.1, 18.2, 19.4, 20.5, 21.3, 22.3, 23.2, 24.5, 24.9, 25.6, 26.4, 27.1, 28.1, 28.4, 30.7, 31.2, 32.1, 33.3, 36.0, 37.0, and 39.2.

In an optional embodiment, the X-ray powder diffraction pattern, expressed in terms of 2θ angles, which are diffraction angles, of the crystal form H of the compound represented by formula (I) provided in the present disclosure is shown in FIG. 9.

The present disclosure provides a method for preparing the crystal form H of the compound represented by formula (I), and the method is selected from the group consisting of the following methods:

method 1

dissolving the compound represented by formula (I) in a solvent 13 and stirring for crystallization, wherein the solvent 13 is selected from the group consisting of an alcohol solvent,
a mixed solvent of an alcohol solvent and water, a ketone solvent, and an ester solvent; optionally, the alcohol solvent is selected from the group consisting of methanol, ethanol, isopropanol, and n-propanol, the ketone solvent is selected from the group consisting of acetone, 2-butanone, and methyl isobutyl ketone, and the ester solvent is ethyl acetate;

method 2

mixing the compound represented by formula (I) with a solvent 14 and slurrying for crystallization, wherein the solvent 14 is selected from the group consisting of water and an ether solvent;

optionally, the ether solvent is selected from the group consisting of methyl tert-butyl ether and isopropyl ether; and

method 3

a) dissolving the compound represented by formula (I) in a solvent 15, and
b) adding a solvent 16 and crystallizing,

wherein the solvent 15 is selected from the group consisting of an alcohol solvent, a ketone solvent, and an ether solvent, and the solvent 16 is selected from the group consisting of a lower alkane, an ether solvent, and water; optionally, the alcohol solvent is selected from the group consisting of isopropanol, the ketone solvent is acetone, the lower alkane is n-heptane, and the ether solvent is tetrahydrofuran, isopropyl ether, or methyl tert-butyl ether.

The present disclosure provides in another aspect a pharmaceutically acceptable salt of the compound represented by formula (I), wherein the pharmaceutically acceptable salt is selected from the group consisting of a hydrochloride, a sulfate, a mesylate, a p-toluenesulfonate, a phosphate, a citrate, and a malate.

In an optional embodiment, the ratio of the compound represented by formula (I) to an acid molecule is selected from the group consisting of about 3:1-1:3; specifically, the ratio may be selected from the group consisting of about 1:1, 1:2, and 1:3.

The present disclosure provides a method for preparing the pharmaceutically acceptable salt of the compound represented by formula (I), the method comprising a step of reacting a free alkali with an acid molecule selected from the group consisting of hydrochloric acid, sulfuric acid, mesylic acid, p-toluenesulfonic acid, phosphoric acid, citric acid, and malic acid.

In an optional embodiment, in the method for preparing the pharmaceutically acceptable salt of the compound represented by formula (I) provided in the present disclosure, the reaction is performed under the action of a solvent.

In an optional embodiment, the solvent is a nitrile solvent, an alcohol solvent, an ester solvent, an aromatic hydrocarbon solvent, an ether solvent, a ketone solvent, or a substituted lower alkane solvent.

In an optional embodiment, the nitrile solvent is acetonitrile.

In an optional embodiment, the alcohol solvent is ethanol.

The pharmaceutically acceptable salt of the compound represented by formula (I) provided in the present disclosure may be in any crystal form or in an amorphous form. The substituted lower alkane described in the present disclosure may be selected from the group consisting of nitromethane, dichloromethane, and trichloromethane; the nitrile solvent is selected from the group consisting of acetonitrile and propionitrile; the alcohol solvent refers to a $C_1$-$C_6$ alcohol and may be selected from the group consisting of methanol, ethanol, n-propanol, isopropanol, and n-butanol; the ketone solvent is selected from the group consisting of acetone, 2-butanone, and methyl isobutyl ketone; the ether solvent is selected from the group consisting of isopropyl ether, tetrahydrofuran, dioxane, and propylene glycol methyl ether; the ester solvent is selected from the group consisting of ethyl acetate, methyl acetate, and isopropyl acetate; the lower alkane is selected from the group consisting of n-hexane and n-heptane.

In the preparation method provided in the present disclosure, the ratio of the solvent may be 0.1-100 times (w/v), specifically 1, 5, 10, 15, 20, 25, 30, 35, 40, 45, 50, 55, 60, 65, 70, 75, 80, 85, 90, 95, or 100 times, or a value between any two of these values, that of the compound represented by formula (I).

The preparation methods for the crystal forms provided in the present disclosure comprise a step of separating a solid from a liquid; specifically, the step may be filtration, centrifugation, or complete volatilization of a solvent.

The preparation methods for the crystal forms provided in the present disclosure optionally comprise a drying step.

The present disclosure also provides a pharmaceutical composition comprising any of the aforementioned crystal forms or pharmaceutically acceptable salts, or a mixture thereof, and optionally a pharmaceutically acceptable auxiliary material selected from the group consisting of pharmaceutically acceptable excipients.

The present disclosure also provides a preparation method for a pharmaceutical composition, the method comprising a step of mixing any of the aforementioned crystal forms or pharmaceutically acceptable salts with a pharmaceutically acceptable excipient.

The present disclosure also provides use of any of the aforementioned crystal forms or pharmaceutically acceptable salts, or the aforementioned composition in the preparation of a medicament for treating or preventing a cyclin-dependent kinase-associated disease.

The present disclosure also provides use of any of the aforementioned crystal forms or pharmaceutically acceptable salts, or the aforementioned composition in the preparation of a medicament for treating or preventing a cancer.

In an optional embodiment, the cancer is selected from the group consisting of breast cancer, ovarian cancer, bladder cancer, uterine cancer, prostate cancer, lung cancer, esophageal cancer, head and neck cancer, intestinal cancer, renal cancer, liver cancer, pancreatic cancer, gastric cancer, and thyroid cancer.

As used herein, "$2\theta$ or $2\theta$ angle" refers to a diffraction angle; $\theta$ refers to the Bragg angle in ° or degrees; the $2\theta$ of each characteristic peak has a margin of error of ±0.2 (including the case that a number having more than 1 decimal place is rounded), and the margin of error may be -0.20, -0.19, -0.18, -0.17, -0.16, -0.15, -0.14, -0.13, -0.12, - 0.11, -0.10, -0.09, -0.08, -0.07, -0.06, -0.05, -0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, or 0.20.

As used herein, the drying temperature is generally 25 °C-100 °C, preferably 40 °C-70 °C, and the drying may be performed under atmospheric pressure or a reduced pressure of <-0.08 MPa.

As used herein, "excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, or emulsifier that has been approved by the U.S. Food and Drug Administration as acceptable for use in humans or livestock animals.

As used herein, "slurrying" refers to a purification method utilizing the low solubility of the target substance and high solubility of the impurities in a solvent, which is capable of decoloring, changing the crystal form, or removing small amounts of impurities.

The starting materials used in the preparation methods for the crystal forms of the present disclosure may be any form of the compound, specifically including, but not limited to: an amorphous form, any crystal form, a hydrate, a solvate, and the like.

In the present disclosure, for the ratio of the compound represented by formula (I) to acid, ±10% is a reasonable margin of error. The margin of error may be ±9%, ±8%, ±7%, ±6%, ±5%, ±4%, ±3%, ±2%, or ±1%.

PCT/CN2022/074509 is incorporated herein by reference in its entirety.

## BRIEF DESCRIPTION OF THE DRAWINGS

FIG. 1 shows an XRPD pattern of the amorphous form of the compound represented by formula (I).
FIG. 2 shows an XRPD pattern of the crystal form A of the compound represented by formula (I).
FIG. 3 shows an XRPD pattern of the crystal form B of the compound represented by formula (I).
FIG. 4 shows an XRPD pattern of the crystal form C of the compound represented by formula (I).
FIG. 5 shows an XRPD pattern of the crystal form D of the compound represented by formula (I).
FIG. 6 shows an XRPD pattern of the crystal form E of the compound represented by formula (I).
FIG. 7 shows an XRPD pattern of the crystal form F of the compound represented by formula (I).
FIG. 8 shows an XRPD pattern of the crystal form G of the compound represented by formula (I).
FIG. 9 shows an XRPD pattern of the crystal form H of the compound represented by formula (I).

## DETAILED DESCRIPTION

The present disclosure is further described below with reference to examples, but these examples are not intended to limit the scope of the present disclosure.

Examples

The structures of the compounds were determined by nuclear magnetic resonance (NMR) spectroscopy or/and mass spectrometry (MS). The NMR shifts (δ) are given in $10^{-6}$ (ppm). The NMR analyses were performed using a Bruker AVANCE NEO 500M nuclear magnetic resonance instrument, with dimethyl sulfoxide-D6 (DMSO-$d_6$), chloroform-D ($CDCl_3$) and methanol-D4 ($CD_3OD$) as solvents and tetramethylsilane (TMS) as an internal standard.

The MS analyses were performed on an Agilent 1200/1290 DAD-6110/6120 Quadrupole MS liquid chromatography-mass spectrometry system (manufacturer: Agilent; MS model: 6110/6120 Quadrupole MS),

waters ACQuity UPLC-QD/SQD (manufacturer: waters; MS model: waters ACQuity Qda Detector/waters SQ Detector), and
THERMO Ultimate 3000-Q Exactive (manufacturer: THERMO; MS model: THERMO Q Exactive).

The high performance liquid chromatography (HPLC) analyses were performed using Agilent HPLC 1200DAD, Agilent HPLC 1200VWD, and Waters HPLC e2695-2489 high-pressure liquid chromatographs.

The chiral HPLC analyses were performed using an Agilent 1260 DAD high performance liquid chromatograph.
The preparative high performance liquid chromatography steps were performed using Waters 2545-2767, Waters 2767-SQ Detecor2, Shimadzu LC-20AP, and Gilson GX-281 preparative chromatographs.
The preparative chiral chromatography steps were performed using a Shimadzu LC-20AP preparative chromatograph.
The CombiFlash preparative flash chromatograph used was Combiflash Rf200 (TELEDYNE ISCO).
The thin-layer chromatography silica gel plates used were Yantai Huanghai HSGF254 or Qingdao GF254 silica gel plates. The silica gel plates used in the thin-layer chromatography (TLC) had a layer thickness of 0.15 mm-0.2 mm, and those used in the thin-layer chromatography separation and purification had a layer thickness of 0.4 mm-0.5 mm.
In the silica gel column chromatography, a 200-300 mesh silica gel (Huanghai, Yantai) was generally used as the carrier.
The kinase mean inhibition rates and $IC_{50}$ values were measured using a NovoStar microplate reader (BMG, Germany).
The known starting materials in the present disclosure may be synthesized by using or following methods known in the art, or may be purchased from companies such as ABCR GmbH & Co. KG, Acros Organics, Aldrich Chemical Company, Accela ChemBio Inc., and Chembee Chemicals.
In the examples, the reactions can all be performed in an argon atmosphere or a nitrogen atmosphere unless otherwise specified.
The argon atmosphere or nitrogen atmosphere means that the reaction flask was connected to a balloon containing about 1 L of argon or nitrogen gas.
The hydrogen atmosphere means that the reaction flask was connected to a balloon containing about 1 L of hydrogen gas.
The pressurized hydrogenation reactions were performed using a Parr 3916EKX hydrogenator and a Qinglan QL-500 hydrogenator, or an HC2-SS hydrogenator.
The hydrogenation reactions generally involved 3 cycles of vacuumization and hydrogen filling.
The microwave reactions were performed using a CEM Discover-S 908860 microwave reactor.
In the examples, the solutions were aqueous solutions unless otherwise specified.
In the examples, the reaction temperature was room temperature, i.e., 20 °C-30 °C, unless otherwise specified.
The monitoring of the reaction progress in the examples was performed by thin-layer chromatography (TLC). The developing solvent for the reactions, the eluent system for the column chromatography purification, and the developing solvent system for thin-layer chromatography include: A: a dichloromethane/methanol system. The volume ratio of the solvents was adjusted depending on the polarity of the compound, or by adding a small amount of basic or acidic reagents such as triethylamine and acetic acid.
Test conditions for the instruments used in the experiments in the present disclosure:

1. Differential scanning calorimeter (DSC)

   Instrument model: Mettler Toledo DSC 3+STARe System
   Gas purging: nitrogen gas; nitrogen purging speed: 50 mL/min
   Ramping rate: 10.0 °C/min
   Temperature range: 25-350 °C or 25-300 °C

2. X-ray powder diffraction (XRPD)

   Instrument model: BRUKER D8 Discover X-ray powder diffractometer
   Rays: monochromatic Cu-K$\alpha$ rays ($\lambda$ = 1.5406)
   Scan mode: $\theta/2\theta$, scan range ($2\theta$ range): 3-45°
   Voltage: 40 kV, current: 40 mA

3. Thermogravimetric analysis (TGA)

   Instrument model: Mettler Toledo TGA2
   Gas purging: nitrogen gas; nitrogen purging speed: 50 mL/min
   Ramping rate: 10.0 °C/min
   Temperature range: 30-350 °C or 30-300 °C

4. DVS refers to dynamic vapor sorption

Testing was performed using Surface Measurement Systems advantage 2, at 25 °C, with the humidity changing from 50% -95%-0%-95%-50%RH, in steps of 10%. The criterion was that each gradient mass change dM/dT is less than 0.002%, TMAX 360 min, two cycles.

Example 1. Preparation of Free-State Amorphous Form

10 mg of the compound represented by formula (I) was added to 0.5 mL of water. The mixture was stirred at room temperature and centrifuged, and drying was then performed *in vacuo* to give a solid. The product was an amorphous form, as identified by X-ray powder diffraction. An XRPD pattern of the amorphous form is shown in FIG. 1.

Example 2. Preparation of Free-State Crystal Form A

10 mg of the compound represented by formula (I) was added to 0.05 mL of isopropyl acetate and completely dissolved. The solution was cooled to 5 °C, stirred for crystallization, and filtered. The filter cake was collected and dried *in vacuo* to give a solid. According to an X-ray powder diffraction analysis, the product was defined as crystal form A. An XRPD pattern of the crystal form is shown in FIG. 2, and the positions of its characteristic peaks are shown in Table 1. A DSC profile of the crystal form shows endothermic peaks at 87.79 °C and 254.08 °C. A TGA profile of the crystal form shows a weight loss of 10.07% from 30 °C to 150 °C.

Table 1

| Peak No. | 2$\theta$ value [° or degrees] | D[Å] | Relative intensity% |
|---|---|---|---|
| 1 | 7.756 | 11.38921 | 52.5 |
| 2 | 9.626 | 9.18041 | 89.3 |
| 3 | 9.975 | 8.86031 | 88.2 |
| 4 | 11.707 | 7.55285 | 94.5 |
| 5 | 15.046 | 5.88360 | 67.1 |
| 6 | 18.862 | 4.70086 | 51.4 |
| 7 | 19.435 | 4.56366 | 52.8 |
| 8 | 20.122 | 4.40938 | 55.5 |
| 9 | 21.114 | 4.20434 | 100.0 |
| 10 | 21.687 | 4.09462 | 90.9 |
| 11 | 23.519 | 3.77966 | 30.4 |
| 12 | 26.686 | 3.33776 | 20.9 |
| 13 | 29.396 | 3.03595 | 48.8 |

Example 3. Preparation of Free-State Crystal Form A

10 mg of the compound represented by formula (I) was added to 0.05 mL of ethyl acetate and completely dissolved. The solution was cooled to 5 °C, stirred for crystallization, and filtered. The filter cake was collected and dried *in vacuo* to give a solid. The product was crystal form A, as identified by X-ray powder diffraction.

Example 4. Preparation of Free-State Crystal Form A

10 mg of the compound represented by formula (I) was added to 0.05 mL of methyl isobutyl ketone and completely dissolved. The solution was cooled to 5 °C, stirred for crystallization, and filtered. The filter cake was collected and dried *in vacuo* to give a solid. The product was crystal form A, as identified by X-ray powder diffraction.

Example 5. Preparation of Free-State Crystal Form A

10 mg of the compound represented by formula (I) was added to 0.05 mL of acetone and dissolved, and 0.6 mL of isopropyl ether was added. The mixture was stirred for crystallization and centrifuged, and drying was then performed *in vacuo* to give a solid. The product was crystal form A, as identified by X-ray powder diffraction.

Example 6. Preparation of Free-State Crystal Form A

10 mg of the compound represented by formula (I) was added to 0.05 mL of methyl isobutyl ketone and dissolved, and 0.6 mL of isopropyl ether was added. The mixture was stirred for crystallization and centrifuged, and drying was then performed *in vacuo* to give a solid. The product was crystal form A, as identified by X-ray powder diffraction.

Example 7. Preparation of Free-State Crystal Form A

10 mg of the compound represented by formula (I) was added to 0.05 mL of ethyl acetate and dissolved, and 0.8 mL of methyl tert-butyl ether was added. The mixture was volatilized for crystallization. The product was crystal form A, as identified by X-ray powder diffraction.

Example 8. Preparation of Free-State Crystal Form B

10 mg of the compound represented by formula (I) was added to 0.05 mL of nitromethane and completely dissolved. The solution was stirred for crystallization at room temperature and filtered. The filter cake was collected and dried *in vacuo* to give a solid. According to an X-ray powder diffraction analysis, the product was defined as crystal form B. An XRPD pattern of the crystal form is shown in FIG. 3, and the positions of its characteristic peaks are shown in Table 2. A DSC profile of the crystal form shows endothermic peaks at 154.21 °C and 254.73 °C. A TGA profile of the crystal form shows a weight loss of 4.42% from 30 °C to 150 °C.

Table 2

| Peak No. | 2$\theta$ value [° or degrees] | D[Å] | Relative intensity% |
|---|---|---|---|
| 1 | 7.422 | 11.90139 | 20.3 |
| 2 | 9.481 | 9.32048 | 4.2 |
| 3 | 11.250 | 7.85898 | 38.0 |
| 4 | 12.444 | 7.10751 | 6.9 |
| 5 | 12.838 | 6.89008 | 8.5 |
| 6 | 13.670 | 6.47241 | 2.3 |
| 7 | 14.303 | 6.18731 | 22.3 |
| 8 | 15.068 | 5.87513 | 100.0 |
| 9 | 16.260 | 5.44683 | 18.5 |
| 10 | 18.008 | 4.92200 | 16.6 |
| 11 | 19.168 | 4.62663 | 9.2 |
| 12 | 19.975 | 4.44136 | 14.8 |
| 13 | 20.884 | 4.25010 | 30.7 |
| 14 | 22.828 | 3.89242 | 29.2 |
| 15 | 23.154 | 3.83829 | 19.1 |
| 16 | 23.684 | 3.75359 | 46.7 |
| 17 | 25.098 | 3.54522 | 19.6 |
| 18 | 26.017 | 3.42212 | 25.1 |
| 19 | 26.755 | 3.32931 | 5.9 |
| 20 | 28.231 | 3.15854 | 5.4 |
| 21 | 30.391 | 2.93880 | 10.8 |
| 22 | 32.738 | 2.73328 | 7.8 |
| 23 | 33.585 | 2.66622 | 3.2 |
| 24 | 34.317 | 2.61102 | 3.5 |

(continued)

| Peak No. | 2$\theta$ value [° or degrees] | D[Å] | Relative intensity% |
|---|---|---|---|
| 25 | 35.473 | 2.52856 | 3.1 |
| 26 | 38.375 | 2.34377 | 4.0 |
| 27 | 39.017 | 2.30666 | 1.8 |
| 28 | 40.481 | 2.22656 | 2.5 |

Example 9. Preparation of Free-State Crystal Form C

10 mg of the compound represented by formula (I) was added to 0.05 mL of dichloromethane and completely dissolved. The solution was volatilized for crystallization to give a solid. According to an X-ray powder diffraction analysis, the product was defined as crystal form C. An XRPD pattern of the crystal form is shown in FIG. 4, and the positions of its characteristic peaks are shown in Table 3. A DSC profile of the crystal form shows endothermic peaks at 125.96 °C and 253.40 °C. A TGA profile of the crystal form shows a weight loss of 4.37% from 30 °C to 150 °C.

Table 3

| Peak No. | 2$\theta$ value [° or degrees] | D[Å] | Relative intensity% |
|---|---|---|---|
| 1 | 4.807 | 18.36762 | 100.0 |
| 2 | 7.573 | 11.66467 | 36.5 |
| 3 | 10.088 | 8.76145 | 50.1 |
| 4 | 11.955 | 7.39689 | 41.8 |
| 5 | 14.965 | 5.91504 | 44.1 |
| 6 | 19.653 | 4.51361 | 61.7 |
| 7 | 21.177 | 4.19205 | 31.8 |
| 8 | 23.501 | 3.78241 | 8.8 |

Example 10. Preparation of Free-State Crystal Form C

10 mg of the compound represented by formula (I) was added to 0.05 mL of 1,2-dichloroethane and completely dissolved. The solution was volatilized for crystallization to give a solid. The product was crystal form C, as identified by X-ray powder diffraction.

Example 11. Preparation of Free-State Crystal Form C

10 mg of the compound represented by formula (I) was added to 0.05 mL of methyl isobutyl ketone and completely dissolved. The solution was volatilized for crystallization to give a solid. The product was crystal form C, as identified by X-ray powder diffraction.

Example 12. Preparation of Free-State Crystal Form C

10 mg of the compound represented by formula (I) was added to 1 mL of p-xylene. The mixture was stirred for crystallization and centrifuged, and drying was then performed *in vacuo* to give a solid. The product was crystal form C, as identified by X-ray powder diffraction.

Example 13. Preparation of Free-State Crystal Form C

10 mg of the compound represented by formula (I) was added to 1 mL of toluene. The mixture was stirred for crystallization and centrifuged, and drying was then performed *in vacuo* to give a solid. The product was crystal form C, as identified by X-ray powder diffraction.

Example 14. Preparation of Free-State Crystal Form C

10 mg of the compound represented by formula (I) was added to 0.1 mL of isopropanol. After 1 mL of isopropyl ether was added, the mixture was stirred to precipitate a solid and centrifuged, and drying was then performed *in vacuo* to give a solid. The product was crystal form C, as identified by X-ray powder diffraction.

Example 15. Preparation of Free-State Crystal Form C

10 mg of the compound represented by formula (I) was added to 0.1 mL of acetone. After 1 mL of isopropyl ether was added, the mixture was stirred to precipitate a solid and centrifuged, and drying was then performed *in vacuo* to give a solid. The product was crystal form C, as identified by X-ray powder diffraction.

Example 16. Preparation of Free-State Crystal Form C

10 mg of the compound represented by formula (I) was added to 0.1 mL of ACN (acetonitrile). After 1 mL of isopropyl ether was added, the mixture was stirred to precipitate a solid and centrifuged, and drying was then performed *in vacuo* to give a solid. The product was crystal form C, as identified by X-ray powder diffraction.

Example 17. Preparation of Free-State Crystal Form C

10 mg of the compound represented by formula (I) was added to 0.1 mL of THF (tetrahydrofuran). After 1 mL of isopropyl ether was added, the mixture was stirred to precipitate a solid and centrifuged, and drying was then performed *in vacuo* to give a solid. The product was crystal form C, as identified by X-ray powder diffraction.

Example 18. Preparation of Free-State Crystal Form C

10 mg of the compound represented by formula (I) was added to 0.1 mL of EtOH. After 1 mL of isopropyl ether was added, the mixture was stirred to precipitate a solid and centrifuged, and drying was then performed *in vacuo* to give a solid. The product was crystal form C, as identified by X-ray powder diffraction.

Example 19. Preparation of Free-State Crystal Form D

10 mg of the compound represented by formula (I) was added to 1 mL of isopropyl ether. The mixture was stirred at 60 °C for 2 h and centrifuged, and drying was then performed *in vacuo* to give a solid. According to an X-ray powder diffraction analysis, the product was defined as crystal form D. An XRPD pattern of the crystal form is shown in FIG. 5, and the positions of its characteristic peaks are shown in Table 4. A DSC profile shows an endothermic peak at 196.66 °C. A TGA profile of the crystal form shows a weight loss of 0.63% from 30 °C to 150 °C.

Table 4

| Peak No. | 2θ value [° or degrees] | D[Å] | Relative intensity% |
|---|---|---|---|
| 1 | 9.976 | 8.85915 | 82.3 |
| 2 | 10.525 | 8.39831 | 61.5 |
| 3 | 11.037 | 8.00993 | 26.7 |
| 4 | 12.510 | 7.06986 | 16.3 |
| 5 | 13.385 | 6.60948 | 28.4 |
| 6 | 15.885 | 5.57479 | 26.4 |
| 7 | 17.016 | 5.20670 | 89.2 |
| 8 | 18.259 | 4.85496 | 23.3 |
| 9 | 18.744 | 4.73029 | 93.9 |
| 10 | 20.093 | 4.41560 | 17.2 |
| 11 | 20.811 | 4.26481 | 19.9 |
| 12 | 22.252 | 3.99187 | 4.3 |

(continued)

| Peak No. | 2θ value [° or degrees] | D[Å] | Relative intensity% |
|---|---|---|---|
| 13 | 23.922 | 3.71690 | 100.0 |
| 14 | 26.733 | 3.33206 | 7.1 |
| 15 | 27.955 | 3.18908 | 25.3 |
| 16 | 30.500 | 2.92852 | 18.3 |
| 17 | 31.006 | 2.88186 | 15.4 |
| 18 | 32.068 | 2.78882 | 4.1 |
| 19 | 33.133 | 2.70155 | 5.0 |
| 20 | 33.780 | 2.65129 | 2.1 |
| 21 | 34.617 | 2.58907 | 5.2 |

Example 20. Preparation of Free-State Crystal Form D

100 mg of the compound represented by formula (I) was added to 10 mL of isopropyl ether, and about 1-2 mg of a seed crystal was added. Slurrying was performed at 60 °C for 2 h. The slurry was centrifuged, and drying was then performed *in vacuo* to give a solid. The product was crystal form D, as identified by X-ray powder diffraction.

Example 21. Preparation of Free-State Crystal Form D

50 mg of the compound represented by formula (I) was added to 0.1 mL of ethanol and completely dissolved by stirring at 60 °C. 0.4 mL of isopropyl ether was added, and 1-2 mg of a seed crystal of crystal form D was added. The mixture was stirred at 60 °C for 2 h to precipitate a solid and centrifuged, and drying was then performed *in vacuo* to give a solid. The product was crystal form D, as identified by X-ray powder diffraction.

Example 22. Preparation of Free-State Crystal Form D

50 mg of the compound represented by formula (I) was added to 0.1 mL of methanol and completely dissolved by stirring at 60 °C. 0.4 mL of isopropyl ether was added, and 1-2 mg of a seed crystal of crystal form D was added. The mixture was stirred at 60 °C for 2 h to precipitate a solid and centrifuged, and drying was then performed *in vacuo* to give a solid. The product was crystal form D, as identified by X-ray powder diffraction.

Example 23. Preparation of Free-State Crystal Form D

700 mg of the compound represented by formula (I) was added to 1.4 mL of ethanol and completely dissolved by stirring at 60 °C. 4.2 mL of isopropyl ether was added, and 1-2 mg of a seed crystal of crystal form D was added. The mixture was stirred at 60 °C for 2 h to precipitate a solid and centrifuged, and drying was then performed *in vacuo* to give a solid. The product was crystal form D, as identified by X-ray powder diffraction.

Example 24. Preparation of Free-State Crystal Form D

10 mg of the compound represented by formula (I) was added to 0.4 mL of ethanol/isopropyl ether (1/20) and completely dissolved by stirring at 60 °C. The solution was stirred for another 1 h to precipitate a solid and centrifuged, and drying was then performed *in vacuo* to give a solid. The product was crystal form D, as identified by X-ray powder diffraction.

Example 25. Preparation of Free-State Crystal Form D

10 mg of the compound represented by formula (I) was added to 0.4 mL of methanol/isopropyl ether (1/20) and completely dissolved by stirring at 60 °C. The solution was stirred for another 1 h to precipitate a solid and centrifuged, and drying was then performed *in vacuo* to give a solid. The product was crystal form D, as identified by X-ray powder diffraction.

Example 26. Preparation of Free-State Crystal Form E

10 mg of the compound represented by formula (I) was added to 0.05 mL of acetonitrile and completely dissolved. The solution was cooled to 5 °C, stirred at the low temperature for crystallization, and filtered. The filter cake was collected and dried *in vacuo* to give a solid. According to an X-ray powder diffraction analysis, the product was defined as crystal form E. An XRPD pattern of the crystal form is shown in FIG. 6, and the positions of its characteristic peaks are shown in Table 5. A DSC profile of the crystal form shows endothermic peaks at 35.46 °C and 137.39 °C. A TGA profile of the crystal form shows a weight loss of 6.04% from 30 °C to 160 °C.

Table 5

| Peak No. | 2$\theta$ value [° or degrees] | D[Å] | Relative intensity% |
|---|---|---|---|
| 1 | 4.774 | 18.49674 | 49.1 |
| 2 | 7.421 | 11.90306 | 21.8 |
| 3 | 9.589 | 9.21568 | 10.9 |
| 4 | 11.391 | 7.76202 | 44.1 |
| 5 | 14.397 | 6.14739 | 61.3 |
| 6 | 15.103 | 5.86161 | 100.0 |
| 7 | 16.395 | 5.40246 | 25.8 |
| 8 | 17.886 | 4.95525 | 11.7 |
| 9 | 19.308 | 4.59326 | 32.3 |
| 10 | 19.928 | 4.45180 | 18.0 |
| 11 | 20.885 | 4.24993 | 18.3 |
| 12 | 22.887 | 3.88245 | 23.3 |
| 13 | 23.548 | 3.77504 | 18.6 |
| 14 | 24.212 | 3.67300 | 7.6 |
| 15 | 25.171 | 3.53521 | 10.1 |
| 16 | 25.913 | 3.43556 | 7.7 |
| 17 | 26.783 | 3.32595 | 5.0 |
| 18 | 30.488 | 2.92964 | 5.5 |

Example 27. Preparation of Free-State Crystal Form E

100 mg of the compound represented by formula (I) was added to 0.5 mL of acetone and completely dissolved, and 3 mL of isopropyl ether was added. The mixture was cooled to 5 °C, stirred for crystallization, and filtered. The filter cake was collected and dried *in vacuo* to give a solid. The product was crystal form E, as identified by X-ray powder diffraction.

Example 28. Preparation of Free-State Crystal Form F

100 mg of the compound represented by formula (I) was added to 0.5 mL of methyl isobutyl ketone and completely dissolved. The solution was stirred for crystallization at room temperature and filtered. The filter cake was collected and dried *in vacuo* to give a solid. According to an X-ray powder diffraction analysis, the product was defined as crystal form F. An XRPD pattern of the crystal form is shown in FIG. 7, and the positions of its characteristic peaks are shown in Table 6. A DSC profile of the crystal form shows endothermic peaks at 32.47 °C and 137.02 °C. A TGA profile of the crystal form shows a weight loss of 6.99% from 30 °C to 160 °C.

Table 6

| Peak No. | 2$\theta$ value [° or degrees] | D[Å] | Relative intensity% |
|---|---|---|---|
| 1 | 4.814 | 18.34323 | 12.7 |

(continued)

| Peak No. | 2$\theta$ value [° or degrees] | D[Å] | Relative intensity% |
|---|---|---|---|
| 2 | 9.133 | 9.67547 | 23.1 |
| 3 | 9.786 | 9.03054 | 4.1 |
| 4 | 11.127 | 7.94541 | 45.9 |
| 5 | 11.405 | 7.75231 | 35.2 |
| 6 | 12.036 | 7.34736 | 5.0 |
| 7 | 13.250 | 6.67688 | 27.5 |
| 8 | 14.300 | 6.18882 | 54.8 |
| 9 | 15.116 | 5.85650 | 100.0 |
| 10 | 15.692 | 5.64281 | 76.3 |
| 11 | 16.582 | 5.34192 | 20.0 |
| 12 | 17.925 | 4.94465 | 15.7 |
| 13 | 19.213 | 4.61592 | 31.2 |
| 14 | 19.994 | 4.43733 | 21.0 |
| 15 | 21.087 | 4.20973 | 23.9 |
| 16 | 22.047 | 4.02856 | 38.0 |
| 17 | 22.521 | 3.94485 | 10.5 |
| 18 | 23.628 | 3.76244 | 16.0 |
| 19 | 24.160 | 3.68075 | 8.1 |
| 20 | 24.557 | 3.62209 | 23.2 |
| 21 | 25.189 | 3.53261 | 3.4 |
| 22 | 25.571 | 3.48080 | 7.8 |
| 23 | 26.103 | 3.41098 | 14.4 |
| 24 | 28.697 | 3.10830 | 1.7 |
| 25 | 30.298 | 2.94757 | 6.3 |
| 26 | 35.026 | 2.55979 | 5.0 |

Example 29. Preparation of Free-State Crystal Form F

100 mg of the compound represented by formula (I) was added to 2 mL of tetrahydrofuran and completely dissolved by stirring at 60 °C, and 10 mL of isopropyl ether and a seed crystal of crystal form F were added. The mixture was stirred at 60 °C for 2 h to precipitate a solid and filtered. The filter cake was collected and dried *in vacuo* to give a solid. The product was crystal form F, as identified by X-ray powder diffraction.

Example 30. Preparation of Free-State Crystal Form G

50 mg of the compound represented by formula (I) was added to 0.1 mL of methanol and 0.4 mL of isopropyl ether solvent and completely dissolved. The solution was stirred at 60 °C for 2 h to precipitate a solid and filtered. The filter cake was collected and dried *in vacuo* to give a solid. According to an X-ray powder diffraction analysis, the product was defined as crystal form G. An XRPD pattern of the crystal form is shown in FIG. 8, and the positions of its characteristic peaks are shown in Table 7. A DSC profile shows an endothermic peak at 173.41 °C. A TGA profile of the crystal form shows a weight loss of 1.18% from 30 °C to 190 °C.

Table 7

| Peak No. | 2$\theta$ value [° or degrees] | D[Å] | Relative intensity% |
|---|---|---|---|
| 1 | 9.129 | 9.67941 | 6.7 |
| 2 | 11.317 | 7.81226 | 57.0 |
| 3 | 12.642 | 6.99656 | 7.3 |
| 4 | 13.780 | 6.42097 | 4.2 |
| 5 | 14.674 | 6.03184 | 14.5 |
| 6 | 15.270 | 5.79766 | 40.0 |
| 7 | 16.076 | 5.50872 | 86.4 |
| 8 | 16.953 | 5.22580 | 8.7 |
| 9 | 17.458 | 5.07581 | 12.9 |
| 10 | 17.944 | 4.93939 | 33.7 |
| 11 | 18.628 | 4.75953 | 100.0 |
| 12 | 19.615 | 4.52221 | 2.8 |
| 13 | 20.771 | 4.27301 | 24.3 |
| 14 | 21.575 | 4.11553 | 13.8 |
| 15 | 22.788 | 3.89922 | 23.9 |
| 16 | 23.134 | 3.84164 | 19.0 |
| 17 | 23.804 | 3.73498 | 18.9 |
| 18 | 24.348 | 3.65274 | 11.0 |
| 19 | 25.286 | 3.51939 | 9.9 |
| 20 | 25.769 | 3.45449 | 14.6 |
| 21 | 27.260 | 3.26887 | 11.1 |
| 22 | 27.806 | 3.20582 | 5.4 |
| 23 | 29.313 | 3.04436 | 4.0 |
| 24 | 30.836 | 2.89736 | 20.3 |
| 25 | 32.288 | 2.77030 | 2.2 |
| 26 | 33.641 | 2.66196 | 2.0 |
| 27 | 34.454 | 2.60100 | 14.6 |
| 28 | 34.955 | 2.56487 | 6.5 |

Example 31. Preparation of Free-State Crystal Form H

10 mg of the compound represented by formula (I) was added to 0.05 mL of ethanol and completely dissolved by stirring and heating at 60 °C. The solution was subjected to suspending, heating, cooling, and stirring processes at 50 °C-5 °C for precipitation and filtered. The filter cake was collected and dried *in vacuo* to give a solid. According to an X-ray powder diffraction analysis, the product was defined as crystal form H. An XRPD pattern of the crystal form is shown in FIG. 9, and the positions of its characteristic peaks are shown in Table 8. ADSC profile shows an endothermic peak at 206.30 °C. A TGA profile of the crystal form shows a weight loss of 0.29% from 33 °C to 218 °C.

Table 8

| Peak No. | 2$\theta$ value [° or degrees] | D[Å] | Relative intensity% |
|---|---|---|---|
| 1 | 10.103 | 8.74836 | 5.5 |
| 2 | 11.672 | 7.57584 | 71.9 |

(continued)

| Peak No. | 2$\theta$ value [° or degrees] | D[Å] | Relative intensity% |
|---|---|---|---|
| 3 | 12.271 | 7.20717 | 24.5 |
| 4 | 12.775 | 6.92411 | 5.8 |
| 5 | 13.371 | 6.61658 | 11.8 |
| 6 | 15.523 | 5.70382 | 100.0 |
| 7 | 17.122 | 5.17472 | 4.6 |
| 8 | 18.165 | 4.87967 | 19.9 |
| 9 | 19.361 | 4.58094 | 69.2 |
| 10 | 20.474 | 4.33427 | 8.2 |
| 11 | 21.318 | 4.16467 | 21.0 |
| 12 | 22.254 | 3.99156 | 22.0 |
| 13 | 23.234 | 3.82533 | 6.8 |
| 14 | 24.547 | 3.62366 | 9.1 |
| 15 | 24.880 | 3.57582 | 19.5 |
| 16 | 25.605 | 3.47621 | 7.4 |
| 17 | 26.366 | 3.37763 | 11.5 |
| 18 | 27.134 | 3.28375 | 13.5 |
| 19 | 28.110 | 3.17192 | 13.3 |
| 20 | 28.421 | 3.13785 | 4.1 |
| 21 | 30.714 | 2.90858 | 7.9 |
| 22 | 31.213 | 2.86322 | 11.6 |
| 23 | 32.143 | 2.78254 | 5.9 |
| 24 | 33.270 | 2.69075 | 2.8 |
| 25 | 35.962 | 2.49530 | 2.6 |
| 26 | 36.976 | 2.42916 | 6.6 |
| 27 | 39.213 | 2.29560 | 3.7 |

Example 32. Preparation of Free-State Crystal Form H

10 mg of the compound represented by formula (I) was added to 0.05 mL of isopropanol and completely dissolved by stirring and heating at 60 °C. The solution was subjected to suspending, heating, cooling, and stirring processes at 50 °C-5 °C for precipitation and filtered. The filter cake was collected and dried *in vacuo* to give a solid. The product was crystal form H, as identified by X-ray powder diffraction.

Example 33. Preparation of Free-State Crystal Form H

10 mg of the compound represented by formula (I) was added to 0.05 mL of n-propanol and completely dissolved by stirring and heating at 60 °C. The solution was subjected to suspending, heating, cooling, and stirring processes at 50 °C-5 °C for precipitation and filtered. The filter cake was collected and dried *in vacuo* to give a solid. The product was crystal form H, as identified by X-ray powder diffraction.

Example 34. Preparation of Free-State Crystal Form H

10 mg of the compound represented by formula (I) was added to 0.05 mL of 2-butanone and completely dissolved by stirring and heating at 60 °C. The solution was subjected to suspending, heating, cooling, and stirring processes at 50 °C-5 °C for precipitation and filtered. The filter cake was collected and dried *in vacuo* to give a solid. The product was crystal

form H, as identified by X-ray powder diffraction.

Example 35. Preparation of Free-State Crystal Form H

10 mg of the compound represented by formula (I) was added to 0.05 mL of a mixed solvent of methanol and water (1/1) and completely dissolved by stirring and heating at 60 °C. The solution was subjected to suspending, heating, cooling, and stirring processes at 50 °C-5 °C for precipitation and filtered. The filter cake was collected and dried *in vacuo* to give a solid. The product was crystal form H, as identified by X-ray powder diffraction.

Example 36. Preparation of Free-State Crystal Form H

10 mg of the compound represented by formula (I) was added to 0.25 mL of ethyl acetate solvent and completely dissolved by stirring and heating at 60 °C. The solution was subjected to suspending, heating, cooling, and stirring processes at 50 °C-5 °C for precipitation and filtered. The filter cake was collected and dried *in vacuo* to give a solid. The product was crystal form H, as identified by X-ray powder diffraction.

Example 37. Preparation of Free-State Crystal Form H

10 mg of the compound represented by formula (I) was added to 0.25 mL of methyl isobutyl ketone solvent and completely dissolved by stirring and heating at 60 °C. The solution was subjected to suspending, heating, cooling, and stirring processes at 50 °C-5 °C for precipitation and filtered. The filter cake was collected and dried *in vacuo* to give a solid. The product was crystal form H, as identified by X-ray powder diffraction.

Example 38. Preparation of Free-State Crystal Form H

10 mg of the compound represented by formula (I) was added to 1 mL of water. The mixture was heated and stirred at 60 °C, and the compound did not dissolve. The mixture was subjected to suspending, heating, cooling, stirring, and slurrying processes at 50 °C-5 °C and filtered. The filter cake was collected and dried *in vacuo* to give a solid. The product was crystal form H, as identified by X-ray powder diffraction.

Example 39. Preparation of Free-State Crystal Form H

10 mg of the compound represented by formula (I) was added to 1 mL of methyl tert-butyl ether. The mixture was heated and stirred at 60 °C, and the compound did not dissolve. The mixture was subjected to suspending, heating, cooling, stirring, and slurrying processes at 50 °C-5 °C and filtered. The filter cake was collected and dried *in vacuo* to give a solid. The product was crystal form H, as identified by X-ray powder diffraction.

Example 40. Preparation of Free-State Crystal Form H

10 mg of the compound represented by formula (I) was added to 1 mL of isopropyl ether. The mixture was heated and stirred at 60 °C, and the compound did not dissolve. The mixture was subjected to suspending, heating, cooling, stirring, and slurrying processes at 50 °C-5 °C and filtered. The filter cake was collected and dried *in vacuo* to give a solid. The product was crystal form H, as identified by X-ray powder diffraction.

Example 41. Preparation of Free-State Crystal Form H

10 mg of the compound represented by formula (I) was added to 0.15 mL of isopropanol and dissolved by stirring at 60 °C. After 1 mL of water was added, the mixture was stirred for precipitation and centrifuged, and drying was then performed *in vacuo* to give a solid. The product was crystal form H, as identified by X-ray powder diffraction.

Example 42. Preparation of Free-State Crystal Form H

10 mg of the compound represented by formula (I) was added to 0.15 mL of isopropanol and dissolved by stirring at 60 °C. After 1 mL of n-heptane was added, the mixture was stirred for precipitation and centrifuged, and drying was then performed *in vacuo* to give a solid. The product was crystal form H, as identified by X-ray powder diffraction.

Example 43. Preparation of Free-State Crystal Form H

10 mg of the compound represented by formula (I) was added to 0.15 mL of isopropanol and dissolved by stirring at 60 °C. After 1 mL of isopropyl ether was added, the mixture was stirred for precipitation and centrifuged, and drying was then performed *in vacuo* to give a solid. The product was crystal form H, as identified by X-ray powder diffraction.

Example 44. Preparation of Free-State Crystal Form H

10 mg of the compound represented by formula (I) was added to 0.1 mL of acetone and dissolved by stirring at 60 °C. After 1 mL of water was added, precipitation was performed. The mixture was centrifuged, and drying was then performed *in vacuo* to give a solid. The product was crystal form H, as identified by X-ray powder diffraction.

Example 45. Preparation of Free-State Crystal Form H

10 mg of the compound represented by formula (I) was added to 0.1 mL of acetone and dissolved by stirring at 60 °C. After 1 mL of n-heptane was added, precipitation was performed. The mixture was centrifuged, and drying was then performed *in vacuo* to give a solid. The product was crystal form H, as identified by X-ray powder diffraction.

Example 46. Preparation of Free-State Crystal Form H

10 mg of the compound represented by formula (I) was added to 0.1 mL of acetone and dissolved by stirring at 60 °C. After 1 mL of isopropyl ether was added, precipitation was performed. The mixture was centrifuged, and drying was then performed *in vacuo* to give a solid. The product was crystal form H, as identified by X-ray powder diffraction.

Example 47. Preparation of Free-State Crystal Form H

10 mg of the compound represented by formula (I) was added to 0.05 mL of tetrahydrofuran and dissolved by stirring at 60 °C. After 1 mL of methyl tert-butyl ether was added, precipitation was performed. The mixture was centrifuged, and drying was then performed *in vacuo* to give a solid. The product was crystal form H, as identified by X-ray powder diffraction.

Example 48. Preparation of Free-State Crystal Form H

10 mg of the compound represented by formula (I) was added to 0.05 mL of tetrahydrofuran and dissolved by stirring at 60 °C. After 1 mL of isopropyl ether was added, precipitation was performed. The mixture was centrifuged, and drying was then performed *in vacuo* to give a solid. The product was crystal form H, as identified by X-ray powder diffraction.

Example 49. Hygroscopicity Study of Free-State Crystal Forms

Surface Measurement Systems intrinsic DVS was adopted. At 25 °C, the crystal forms were tested in a humidity range of 0%-95%, with increments of 10%. The criterion was that each gradient mass change dM/dT is less than 0.002%, TMAX 360 min, two cycles.

Table 9

| Crystal form | 0.0% RH-80.0% RH | Crystal form |
|---|---|---|
| Crystal form D | 0.46% | No change |
| Crystal form E | 3.45% | No change |
| Crystal form F | 1.10% | No change |
| Crystal form G | 2.46% | No change |
| Crystal form H | 0.28% | No change |

Example 50. Influencing Factor Stability Study of Crystal Forms Free-state samples were spread out, left to stand in open flasks, and subjected to 30-day stability tests under light exposure (4500 Lux), high temperature (40 °C and 60 °C), and high humidity (RH 75% and RH 92.5%) conditions.

Table 10

| Conditions | Time (days) | Purity% | Crystal form |
|---|---|---|---|
| Initial | 0 | 99.9 | D |
| Light exposure (4500 Lux) | 7 | 96.0 | D |
| | 14 | 94.6 | D |
| | 30 | 92.8 | D |
| 40 °C | 7 | 99.9 | D |
| | 14 | 99.9 | D |
| | 30 | 99.9 | D |
| 60 °C | 7 | 99.9 | D |
| | 14 | 99.9 | D |
| | 30 | 99.9 | D |
| 75% RH | 7 | 99.9 | D |
| | 14 | 99.9 | D |
| | 30 | 99.9 | D |
| 92.5% RH | 7 | 99.9 | D |
| | 14 | 99.9 | D |
| | 30 | 99.9 | D |

Table 11

| Conditions | Time (days) | Purity% | Crystal form |
|---|---|---|---|
| Initial | 0 | 99.9 | E |
| Light exposure (4500 Lux) | 7 | 99.6 | E |
| | 14 | 99.3 | E |
| | 30 | 98.7 | E |
| 40 °C | 7 | 99.9 | E |
| | 14 | 99.8 | E |
| | 30 | 99.8 | E |
| 60 °C | 7 | 99.9 | E |
| | 14 | 99.9 | E |
| | 30 | 99.9 | E |
| 75% RH | 7 | 99.9 | E |
| | 14 | 99.8 | E |
| | 30 | 99.8 | E |
| 92.5% RH | 7 | 99.9 | E |
| | 14 | 99.8 | E |
| | 30 | 99.8 | E |

Table 12

| Conditions | Time (days) | Purity% | Crystal form |
|---|---|---|---|
| Initial | 0 | 99.9 | F |

(continued)

| Conditions | Time (days) | Purity% | Crystal form |
|---|---|---|---|
| Light exposure (4500 Lux) | 7 | 99.6 | F |
| | 14 | 99.4 | F |
| | 30 | 98.1 | F |
| 40 °C | 7 | 99.9 | F |
| | 14 | 99.9 | F |
| | 30 | 99.9 | F |
| 60 °C | 7 | 99.9 | F |
| | 14 | 99.9 | F |
| | 30 | 99.9 | F |
| 75% RH | 7 | 99.9 | F |
| | 14 | 99.9 | F |
| | 30 | 99.9 | F |
| 92.5% RH | 7 | 99.9 | F |
| | 14 | 99.9 | F |
| | 30 | 99.9 | F |

Table 13

| Conditions | Time (days) | Purity% | Crystal form |
|---|---|---|---|
| Initial | 0 | 99.9 | G |
| Light exposure (4500 Lux) | 7 | 98.5 | G |
| | 14 | 98.1 | G |
| | 30 | 96.8 | G |
| 40 °C | 7 | 99.9 | G |
| | 14 | 99.9 | G |
| | 30 | 99.9 | G |
| 60 °C | 7 | 99.9 | G |
| | 14 | 99.9 | G |
| | 30 | 99.9 | G |
| 75% RH | 7 | 99.9 | G |
| | 14 | 99.9 | G |
| | 30 | 99.9 | G |
| 92.5% RH | 7 | 99.9 | G |
| | 14 | 99.9 | G |
| | 30 | 99.9 | G |

Table 14

| Conditions | Time (days) | Purity% | Crystal form |
|---|---|---|---|
| Initial | 0 | 99.0 | Free-state amorphous form |

(continued)

| Conditions | Time (days) | Purity% | Crystal form |
|---|---|---|---|
| Light exposure (4500 Lux) | 7 | 98.3 | No change |
| | 14 | 97.8 | No change |
| | 30 | 97.4 | No change |
| 40 °C | 7 | 99.0 | No change |
| | 14 | 99.0 | No change |
| | 30 | 98.9 | No change |
| 60 °C | 7 | 99.0 | No change |
| | 14 | 99.0 | No change |
| | 30 | 99.0 | No change |
| 75% RH | 7 | 99.0 | No change |
| | 14 | 99.0 | No change |
| | 30 | 98.9 | No change |
| 92.5% RH | 7 | 99.0 | No change |
| | 14 | 99.0 | No change |
| | 30 | 98.9 | No change |

**Table 15**

| Conditions | Time (days) | Purity% | Crystal form |
|---|---|---|---|
| Initial | 0 | 99.95 | H |
| Light exposure (4500 Lux) | 7 | 99.89 | H |
| | 14 | 99.83 | H |
| | 30 | 99.69 | H |
| 40 °C | 7 | 99.96 | H |
| | 14 | 99.94 | H |
| | 30 | 99.93 | H |
| 60 °C | 7 | 99.95 | H |
| | 14 | 99.94 | H |
| | 30 | 99.92 | H |
| 75% RH | 7 | 99.96 | H |
| | 14 | 99.95 | H |
| | 30 | 99.95 | H |
| 92.5% RH | 7 | 99.95 | H |
| | 14 | 99.95 | H |
| | 30 | 99.94 | H |

The results of the influencing factor experiments show that: crystal forms D/E/F/G/H and the free-state amorphous form exhibited good chemical stability under high temperature and high humidity conditions; crystal forms D/E/F/G/H and the free-state amorphous form exhibited good physical stability.

Example 51. Long-Term/Accelerated Stability of Crystal Forms

Free-state samples were sealed in aluminum foil bags and left to stand under 25 °C/60% RH and 40 °C/75% RH conditions to test their stability. The results are shown below.

Table 16

| | Standing conditions | Purity% | | | | | | Crystal form |
|---|---|---|---|---|---|---|---|---|
| Crystal form D | | Initial | 14 days | 1 month | 2 months | 3 months | 6 months | |
| | 25 °C/60% RH | 99.9 | 99.9 | 99.9 | 99.9 | 99.9 | 99.9 | No change |
| | 40 °C/75% RH | | 99.9 | 99.9 | 99.9 | 99.9 | 99.9 | No change |
| | Standing conditions | Purity% | | | | | | Crystal form |
| Crystal form E | | Initial | 14 days | 1 month | 2 months | 3 months | 6 months | |
| | 25 °C/60% RH | 99.9 | 99.9 | 99.9 | 99.9 | 99.9 | 99.9 | No change |
| | 40 °C/75% RH | | 99.9 | 99.9 | 99.9 | 99.9 | 99.9 | No change |
| | Standing conditions | Purity% | | | | | | Crystal form |
| Crystal form F | | Initial | 14 days | 1 month | 2 months | 3 months | 6 months | |
| | 25 °C/60% RH | 99.9 | 99.9 | 99.9 | 99.9 | 99.9 | 99.9 | No change |
| | 40 °C/75% RH | | 99.9 | 99.9 | 99.9 | 99.9 | 99.9 | No change |
| | Standing conditions | Purity% | | | | | | Crystal form |
| Crystal form G | | Initial | 14 days | 1 month | 2 months | 3 months | 6 months | |
| | 25 °C/60% RH | 99.9 | 99.9 | 99.9 | 99.9 | 99.9 | 99.9 | No change |
| | 40 °C/75% RH | | 99.9 | 99.9 | 99.9 | 99.9 | 99.9 | No change |
| | Standing conditions | Purity% | | | | | | Crystal form |
| Free-state amorphous form | | Initial | 14 days | 1 month | 2 months | 6 months | | |
| | 25 °C/60% RH | 99.0 | 99.0 | 99.0 | 98.9 | 98.8 | | No change |
| | 40 °C/75% RH | | 99.0 | 99.0 | 98.9 | 98.8 | | Deliquescence |
| | Standing conditions | Purity % | | | | | | Crystal form |
| Crystal form H | | Initial | 14 days | 1 month | 2 months | 3 months | | |
| | 25 °C/60% RH | 99.95 | 99.95 | 99.95 | 99.95 | 99.95 | | No change |
| | 40 °C/75% RH | | 99.95 | 99.95 | 99.95 | 99.95 | | No change |

The experimental results show that: standing under long-term accelerated conditions for 3 months, crystal form H exhibited good physical stability and chemical stability; standing under long-term accelerated conditions for 6 months, crystal form D, crystal form E, crystal form F, and crystal form G exhibited good physical stability and chemical stability; the deliquescence of the free-state amorphous form was accelerated, and the free-state amorphous form exhibited good physical stability and chemical stability under other conditions.

Example 52. Preparation of Hydrochloride

100 mg of the compound represented by formula (I) was added to 2 mL of acetonitrile, and 115.3 μL of a 2 M solution of hydrochloric acid in ethanol was added. After 5 h of stirring, the sample dissolved. 5 mL of isopropyl ether was added, and the mixture was stirred for precipitation and centrifuged. Drying was performed *in vacuo* to give a solid. Ion chromatography results showed a chloride ion content of 6.7%.

Example 53. Preparation of Sulfate

100 mg of the compound represented by formula (I) was added to 2 mL of acetonitrile, and 115.3 μL of a 2 M solution of sulfuric acid in ethanol was added. After 5 h of stirring, the sample dissolved. 5 mL of isopropyl ether was added, and the mixture was stirred for precipitation and centrifuged. Drying was performed *in vacuo* to give a solid. Ion

chromatography results showed a sulfate ion content of 14.9%.

Example 54. Preparation of Mesylate

100 mg of the compound represented by formula (I) was added to 2 mL of acetonitrile, and 115.3 µL of a 2 M solution of mesylic acid in ethanol was added. After 5 h of stirring, the sample dissolved. 5 mL of isopropyl ether was added, and the mixture was stirred for precipitation and centrifuged. Drying was performed *in vacuo* to give a solid. Ion chromatography results showed a mesylate ion content of 17.8%.

Example 55. Preparation of p-Toluenesulfonate

100 mg of the compound represented by formula (I) was added to 2 mL of acetonitrile, and 115.3 µL of a 2 M solution of p-toluenesulfonic acid in ethanol was added. After 5 h of stirring, the sample dissolved. 5 mL of isopropyl ether was added, and the mixture was stirred for precipitation and centrifuged. Drying was performed *in vacuo* to give a solid. Ion chromatography results showed a p-toluenesulfonate ion content of 34.3%.

Example 56. Preparation of Phosphate

10 mg of the compound represented by formula (I) was added to 0.2 mL of acetonitrile, and 11.5 µL of a 2 M solution of phosphoric acid in ethanol was added. The mixture was stirred for 5 h and centrifuged, and drying was performed *in vacuo* to give a solid.

Example 57. Preparation of Citrate

10 mg of the compound represented by formula (I) was added to 0.2 mL of acetonitrile, and 11.5 µL of a 2 M solution of citric acid in ethanol was added. The mixture was stirred for 5 h and centrifuged, and drying was performed *in vacuo* to give a solid.

Example 58. Preparation of Malate

10 mg of the compound represented by formula (I) was added to 0.2 mL of acetonitrile, and 11.5 µL of a 2 M solution of citric acid in ethanol was added. The mixture was stirred for 5 h and centrifuged, and drying was performed *in vacuo* to give a solid.

Example 59.

(3S,4R)-4-((5-Chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)tetrahydro-2H-pyran-3-ol Isomer 1

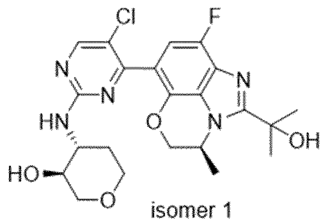

isomer 1

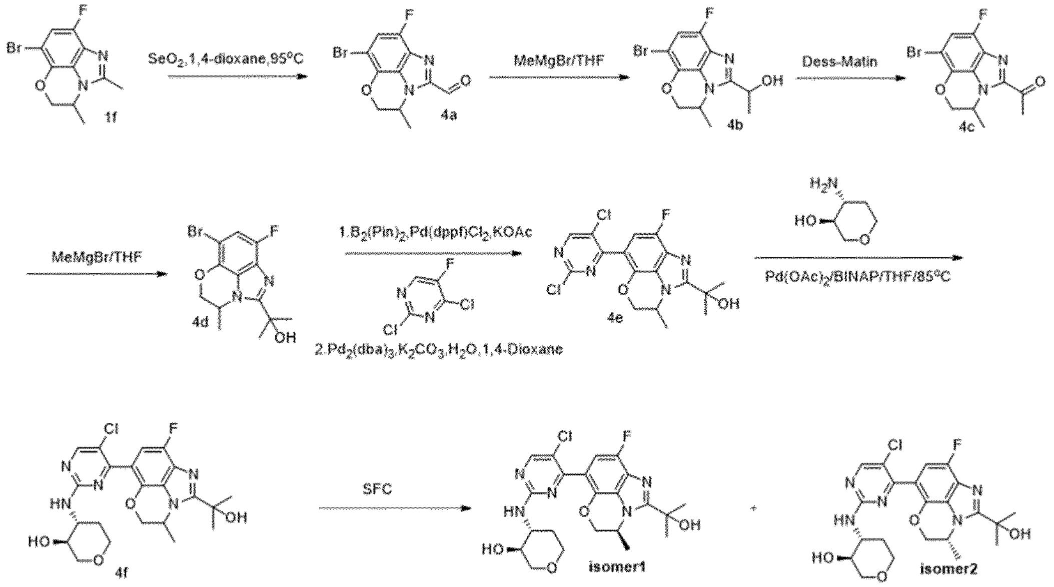


1f, 4a, 4b, 4c, 4d, 4e, 4f, isomer1, isomer2

Step 1

6-Bromo-8-fluoro-3-methyl-3,4-dihydro-5-oxa-1,2a-diazaacenaphthylene-2-carbaldehyde **4a**

In a nitrogen atmosphere, 6-bromo-8-fluoro-2,3-dimethyl-3,4-dihydro-5-oxa-1,2a-diazaacenaphthylene (2.0 g, 7 mmol) and selenium dioxide (3.1 g, 28 mmol) were sequentially added to 30 mL of 1,4-dioxane. The mixture was left to react at 95 °C for 8 h. The reaction mixture was cooled to room temperature and filtered. The filtrate was collected and concentrated under reduced pressure, and the residue was then purified by C-18 reversed-phase chromatography to give title compound **4a** (930 mg, yield: 44%).
MS (ESI) m/z 299.1, 301.1 $[M+H]^+$

Step 2

1-(6-Bromo-8-fluoro-3-methyl-3,4-dihydro-5-oxa-1,2a-diazaacenaphthylen-2-yl)ethan-1-ol **4b**

In a nitrogen atmosphere, 6-bromo-8-fluoro-3-methyl-3,4-dihydro-5-oxa-1,2a-diazaacenaphthylene-2-carbaldehyde (930 mg, 3.1 mmol) was dissolved in 20 mL of tetrahydrofuran. The solution was cooled to -20 °C, and a solution of methylmagnesium bromide in tetrahydrofuran (3 mol/L, 1.5 mL, 4.5 mmol) was added dropwise. The mixture was left to react at -20 °C for 4 h. The reaction was quenched with 5 mL of water. The reaction mixture was concentrated under reduced pressure, and the residue was then purified by C-18 reversed-phase chromatography to give title compound **4b** (830 mg, yield: 85%).
MS (ESI) m/z 315.2, 317.2 $[M+H]^+$

Step 3

1-(6-Bromo-8-fluoro-3-methyl-3,4-dihydro-5-oxa-1,2a-diazaacenaphthylen-2-yl)ethan-1-one **4c**

At room temperature, 1-(6-bromo-8-fluoro-3-methyl-3,4-dihydro-5-oxa-1,2a-diazaacenaphthylen-2-yl)ethan-1-one (600 mg, 1.9 mmol) was dissolved in 20 mL of tetrahydrofuran, and Dess-Martin periodinane (2.0 g, 4.8 mmol) was added. The mixture was heated to 80 °C and left to react for 2 h. The reaction mixture was cooled to room temperature and filtered. The filtrate was collected and concentrated under reduced pressure, and the residue was then purified by C-18

reversed-phase chromatography to give title compound **4c** (350 mg, yield: 59%).
MS (ESI) m/z 313.1, 315.1 $[M+H]^+$

Step 4

2-(6-Bromo-8-fluoro-3-methyl-3,4-dihydro-5-oxa-1,2a-diazaacenaphthylen-2-yl)propan-2-ol **4d**

In a nitrogen atmosphere, 1-(6-bromo-8-fluoro-3-methyl-3,4-dihydro-5-oxa-1,2a-diazaacenaphthylen-2-yl) ethan-1-one (350 mg, 1.1 mmol) was dissolved in 20 mL of tetrahydrofuran. The solution was cooled to -20 °C, and a solution of methylmagnesium bromide in tetrahydrofuran (3 mol/L, 0.7 mL, 2.1 mmol) was added dropwise. The mixture was left to react at -20 °C for 4 h. The reaction was quenched with 5 mL of water. The reaction mixture was concentrated under reduced pressure, and the residue was purified by C-18 reversed-phase chromatography to give title compound **4d** (260 mg, yield: 71%).
MS (ESI) m/z 329.2, 331.2 $[M+H]^+$

Step 5

2-(6-(2,5-Dichloropyrimidin-4-yl)-8-fluoro-3-methyl-3,4-dihydro-5-oxa-1,2a-diazaacenaphthylen-2-yl)propan-2-ol **4e**

In a nitrogen atmosphere, the compounds 2-(6-bromo-8-fluoro-3-methyl-3,4-dihydro-5-oxa-1,2a-diazaacenaphthylen-2-yl)propan-2-ol (260 mg, 0.8 mmol), bis(pinacolato)diboron (305 mg, 1.2 mmol), potassium acetate (157 mg, 1.6 mmol), and [1,1'-bis(diphenylphosphino)ferrocene]palladium(II) dichloride (117 mg, 0.2 mmol) were sequentially dissolved in 5 mL of 1,4-dioxane. The mixture was left to react at 100 °C for 2 h. The reaction mixture was cooled to room temperature, and 1,3,5,7-tetramethyl-6-phenyl-2,4,8-trioxa-6-phosphaadamantane (58 mg, 0.2 mmol), potassium carbonate (221 mg, 1.6 mmol), 2,4-dichloro-5-fluoropyrimidine (220 mg, 1.2 mmol), tris(dibenzylideneacetone)dipalladium(0) (183 mg, 0.2 mmol), and 1 mL of water were added. The mixture was left to react at 80 °C for 1 h. The reaction mixture was cooled to room temperature and filtered. The filtrate was collected and concentrated under reduced pressure, and the residue was then purified by C-18 reversed-phase chromatography to give title compound **4e** (129 mg, yield: 41%).
MS (ESI) m/z 397.3 $[M+H]^+$

Step 6

(3S,4R)-4-((5-Chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)tetrahydro-2H-pyran-3-ol **isomer 1**

(3S,4R)-4-((5-Chloro-4-((R)-8-fluoro-2-(2-hydroxypropan-2-yl)-3-methyl-3,4-dihydro-5-oxa-1,2a-diazaacenaphthylen-6-yl)pyrimidin-2-yl)amino)tetrahydro-2H-pyran-3-ol **isomer 2**

In a nitrogen atmosphere, 2-(6-(2,5-dichloropyrimidin-4-yl)-8-fluoro-3-methyl-3,4-dihydro-5-oxa-1,2a-diazaacenaphthylen-2-yl)propan-2-ol (124 mg, 0.31 mmol), (3S,4R)-4-aminotetrahydro-2H-pyran-3-ol (19 mg, 0.16 mmol), (S)-(-)-2,2"-bis(diphenylphosphino)-1,1"-binaphthyl (118 mg, 0.5 mmol), and palladium acetate (14 mg, 0.06 mmol) were sequentially dissolved in 5 mL of tetrahydrofuran. Cesium carbonate (202 mg, 0.62 mmol) was added, and the mixture was left to react at 85 °C for 3 h. The reaction mixture was cooled to room temperature and filtered. The filtrate was collected and concentrated under reduced pressure, and the residue was then purified by C-18 reversed-phase chromatography to give crude mixture 4f. The crude product was chirally resolved [column:
DAICEL CHIRALPAK AD (250 mm × 30 mm,10 μm); condition: 0.1% $NH_3 \cdot H_2O$ ETOH, Begin B: 40%; End B: 40%; FlowRate (mL/min): 60)] to give title compound isomer 1 (25.2 mg, yield: 17%) and title compound **isomer 2** (22 mg, yield: 15%).

Analysis method

Column: DAICEL CHIRALCEL AD-3 (100 mm × 4.6 mm, 3 μm);
Mobile phase: A: $CO_2$ B: ethanol (0.05% DEA), Gradient: from 5% to 40% of B in 2 min and hold 40% for 1.2 min, then 5% of B for 0.8 min;
FlowRate: 4 mL/min;
ABPR: 1500 psi;

Temperature: 35 °C.

The compound with a retention time of 1.887 min was defined as **isomer 2;**

MS (ESI) m/z 478.1 [M+H]+
$^1$H NMR (400MHz, DMSO-d6) δ = 8.37 (s, 1H), 7.45 (br s, 1H), 6.92 (d, *J*=11.3 Hz, 1H), 5.82 (br s, 1H), 5.23 (q, *J*=6.4 Hz, 1H), 4.47 (d, *J*=11.0 Hz, 1H), 4.22 (br d, *J*=11.3 Hz, 1H), 3.89 - 3.67 (m, 3H), 3.39 - 3.25 (m, 4H), 3.03 (br t, *J*=10.2 Hz, 1H), 1.95 (br d, *J*=10.5 Hz, 1H), 1.68 (s, 3H), 1.62 (s, 3H), 1.45 (d, *J*=6.5 Hz, 3H)

The compound with a retention time of 2.078 min was defined as **isomer 1;**

MS (ESI) m/z 478.1 $[M+H]^+$
$^1$H NMR (400MHz, DMSO-d6) δ = 8.37 (s, 1H), 7.45 (br s, 1H), 6.92 (d, *J*=11.5 Hz, 1H), 5.82 (s, 1H), 5.29 - 5.19 (m, 1H), 4.93 (d, *J*=5.5 Hz, 1H), 4.47 (d, *J*=11.3 Hz, 1H), 4.21 (br d, *J*=10.3 Hz, 1H), 3.85 - 3.75 (m, 3H), 3.53 - 3.40 (m, 2H), 3.03 (br t, *J*=10.4 Hz, 1H), 1.94 (br s, 1H), 1.67 (s, 3H), 1.62 (s, 3H), 1.45 (d, *J*=6.5 Hz, 3H), 1.06 (t, *J=7.0* Hz, 1H)

**Biological Evaluations**

The present invention is further described below using test examples, but these examples are not intended to limit the scope of the present invention.

Test Example 1. Cyclin-Dependent Kinase Activity Assay of Disclosed Compounds

1. Exprimental materials

| Reagent | Manufacturer | Cat. No. |
|---|---|---|
| CDK4/CyclinD1 kinase activity, CDK6/CyclinD3 kinase activity | | |
| CDK4/Cyclin D1 | ProQinase | 0142-0143-1 |
| CDK6/Cyclin D3 | Carna | 04-107 |
| Peptide FAM-P18 | GL Biochem | P080319-XY114202 |
| Peptide FAM-P8 | GL Biochem | P100804-XZ112396 |
| ATP | Sigma | A7699-1G |
| DMSO | Sigma | 474382 |
| EDTA | Sigma | E5134 |
| Staurosporine<br>Ribociclib<br>Compound A | Selleckchem<br>MCE<br>In-house synthesis | S142105<br>HY-15777<br>Compound A |
| CDK1/CyclinB kinase activity, CDK9/CyclinT1 kinase activity | | |
| ADP-Glo Kinase Assay<br>CDK1/CyclinB<br>CDK9/CyclinT1<br>PHA-793887<br>Histone H1 Protein<br>ATP<br>DMSO | Promega<br>proqinase<br>Wuxi Biortus<br>Selleck<br>SignalChem<br>Promega<br>Sigma | V9102<br>0134-0135-1<br>BP480/792/691<br>S1487<br>H10-54N<br>V910B<br>D8418 |

Compound A is Example A94 of WO 2019/207463A1 and was synthesized with reference to the method disclosed in the patent.

2. Kinase activity assay (CDK4/CyclinD1, CDK6/CyclinD3) Mobility shift assay for measuring kinase activity

*In vitro* CDK kinase activity was tested using a mobility shift assay. In the experiment, the starting concentration of the test compounds for the inhibition of CDK activity was 300 nM and was 3-fold diluted to a total of 10 concentrations, and each concentration was tested in duplicate. The compound Staurosporine was used as a standard control. A 1× kinase buffer (CDK2) (50 mM HEPES, pH 7.5, 0.0015% Brij-35), a 1× kinase buffer (CDK4) (20 mM HEPES, pH 7.5, 0.01% Triton X-100), and a stop solution (100 mM HEPES, pH 7.5, 0.015% Brij-35, 0.2% Coating Reagent #3, 50 mM EDTA) were prepared. A proper amount of kinase was added to the 1× kinase buffer to prepare a 2.5× enzyme solution. 5× compound dilutions (1× kinase buffer, 10% DMSO) corresponding to the test concentrations of the compounds were prepared. A proper amount of FAM-labeled polypeptide and ATP was added to the 1× kinase buffer to prepare a 2.5× substrate solution. 5 μL of 5× compound dilution and 10 μL of 2.5× enzyme solution were added to reaction wells of a 384-well reaction plate. After thorough mixing, the plate was incubated at room temperature for 10 min. Then 10 μL of 2.5× substrate solution was added to the 384-well plate, and the plate was centrifuged at 1000 rpm for 1 min. The reaction plate was incubated at 28 °C for 60 min (biochemical incubator model: SPX-100B-Z). 30 μL of stop solution was added to the 384-well reaction plate to stop the reactions, and the plate was centrifuged at 1000 rpm for 1 min. Finally, conversion rate data were obtained on Caliper EZ Reader II (excitation wavelength: 400 nm; emission wavelengths: 445 nm and 520 nm).
$IC_{50}$ values for the compounds were obtained by fitting using XLFit excel add-in version 5.4.0.8. Fitting formula:

$$Y = Bottom + (Top - Bottom) / (1 + (IC50 / X)^{\wedge}HillSlope).$$

3. Kinase activity assay (CDK1/CyclinB, CDK9/CyclinT1)

The starting concentration for the *in vitro* CDK (CDK2 and CDK9) kinase activity assay was 1 μM and was 3-fold diluted to a total of 10 concentrations, and each concentration was tested in duplicate. The compound PHA-793887 was used as a control compound.
A 1× kinase reaction buffer (40 mM Tris-HCl, pH 7.4, 20 mM Mg2Cl2, 0.1 mg/mL BSA, 50 μM DTT), 1 volume of 5× kinase reaction buffer, and 4 volumes of water were prepared, and DTT (final concentration: 50 μM) was added. 50 nL of diluted compound working solution (final concentration of DMSO: 1%) was transferred to each well of a reaction plate (784075, Greiner) using Echo655. The reaction plate was sealed with a plate sealing film and centrifuged at 1000 g for 1 min. 2× enzyme (0.3 ng/μL CDK2/CyclinE1 or CDK9/CyclinT1) was prepared using the 1× kinase reaction buffer. 2.5 μL of the above kinase solution was added to each well. The reaction plate was sealed with a plate sealing film, centrifuged at 1000 g for 1 min, and left to stand at room temperature for 10 min. A mixture of 2× kinase substrate and ATP was prepared using the 1× kinase reaction buffer. 2× CDK2/CylinE1 kinase substrate was 0.4 mg/mL Histone H1 and 30 μM ATP. 2.5 μL of the mixture of 2× Histone H1 and ATP was added to the reaction plate. The plate was centrifuged at 1000 g for 30 s, and the reaction was started. After the kinase assay reaction was carried out at room temperature for 120 min, 4 μL of ADP-Glo reagent was added, and the mixture was left to react at room temperature for 40 min. Then 8 μL of kinase assay reagent was added, and the mixture was left to react at room temperature for 40 min. The luminescence signals were obtained using Envision 2104.
Data analysis is shown below:

a) Percent inhibition: % inhibition = 100 - (Signalcmpd - SignalAve_PC) / (SignalAve_VC - SignalAve_PC) × 100.

SignalAve_PC: an average value for all positive control wells in the whole plate.
SignalAve_VC: an average value for all negative control wells in the whole plate.
b) Compound IC50: obtained using GraphPad 8.0 and the following non-linear fitting formula:

$$Y = Bottom + (Top - Bottom) / (1 + 10^{\wedge}((LogIC50 - X)*HillSlope))$$

X: the log value of the compound concentration; Y: the percent inhibition for the compound
The biochemical inhibitory activity of the compounds of the present disclosure against CDKs (CDK1, CDK4, CDK6, and CDK9) was determined through the above assay. The determined $IC_{50}$ values are shown in Tables 17 and 18.

Table 17.

| Compound | CDK4/D1 (nM) | CDK6/D3 (nM) |
|---|---|---|
| Ribociclib | 27 | 77 |

(continued)

| Compound | CDK4/D1 (nM) | CDK6/D3 (nM) |
|---|---|---|
| Example (**racemate**) | 15 | 171 |

Table 18.

| Compound | CDK1/B (nM) | CDK4/D1 (nM) | CDK6/D3 (nM) | CDK9/T1 (nM) |
|---|---|---|---|---|
| Compound A | 325 | 4.8 | 121 | 190 |
| Isomer 2 with a retention time of 1.887 min | >1000 | 14 | 228 | >1000 |
| Isomer 1 with a retention time of 2.078 min | >1000 | 8.2 | 105 | >1000 |

Test Example 2. CYP Inhibition Assay

The metabolic reactions of representative substrates of 5 major human CYP subtypes (CYP1A2, CYP2C9, CYP2C19, CYP2D6, CYP3A4) were evaluated using 150-donor pooled human liver microsomes (purchased from Corning, Cat. No. 452117). The effects of different concentrations of the test compounds on the metabolic reactions of phenacetin (CYP1A2), diclofenac sodium (CYP2C9), S-mephenytoin (CYP2C19), bufuralol hydrochloride (CYP2D6), and midazolam (CYP3A4/5) were assessed by liquid chromatography-tandem mass spectrometry (LC/MS/MS).

30 μM phenacetin, 10 μM diclofenac sodium, 35 μM S-mephenytoin, 5 μM bufuralol hydrochloride, 3 μM midazolam, 1 mM NADPH, and the test compounds (at concentrations of 0.1 μmol/L, 0.3 μmol/L, 1 μmol/L, 3 μmol/L, 10 μmol/L, and 30 μmol/L, respectively) or the positive compound or the blank control were incubated with 200 μL of a reaction system of pooled human liver microsomes (0.2 mg/mL) (a 100 mmol/L phosphate buffer, pH 7.4, containing 0.3% by volume of DMSO, 0.6% by volume of acetonitrile, and 0.1% by volume of methanol) at 37 °C for 5 min. Then, 200 μL of an acetonitrile solution containing 3% formic acid and 40 nM internal standard verapamil was added, and the mixture was centrifuged at 4000 rpm for 50 min. The mixture was cooled on ice for 20 min and centrifuged at 4000 rpm for 20 min to precipitate protein. 200 μL of the supernatant was taken for LC/MS/MS analysis.

The peak area was calculated based on the chromatogram. The residual activity proportion (%) was calculated using the following formula:

$$\text{peak area ratio} = \text{peak area of metabolite} / \text{peak area of internal standard}$$

residual activity ratio (%) = peak area ratio of test compound group / peak area ratio of blank group

The half maximal inhibitory concentrations ($IC_{50}$) against CYP were calculated using Excel XLfit 5.3.1.3.

The determined half maximal inhibitory concentrations ($IC_{50}$) values against CYP are shown in Table 19 below.

Table 19. The half maximal inhibitory concentrations ($IC_{50}$) of the compounds of the present disclosure against CYP

| Example No. | CYP 1A2 (μM) | CYP 2C9 (μM) | CYP 2C19 (μM) | CYP 2D6 (μM) | CYP 3A4 (μM) |
|---|---|---|---|---|---|
| Compound A | >30 | 22 | >30 | >30 | 16 |
| Isomer 2 with a retention time of 1.887 min | >30 | >30 | >30 | >30 | >30 |
| Isomer 1 with a retention time of 2.078 min | >30 | >30 | >30 | >30 | >30 |

Test Example 3. Solubility Testing

The thermodynamic solubility of the compounds in a pH 7.4 phosphate buffer was determined. Both the sample supernatant and the standard of a known concentration were tested by LC/MS/MS.

1. Materials and reagents

Compound A (compound A is Example A94 of WO 2019/207463A1 and was synthesized with reference to

the method disclosed in the patent).
$NaH_2PO_4 \cdot 2H_2O$ (analytical reagent), $NaH_2PO_4$ (analytical reagent), and NaOH (analytical reagent).
1.5 mL flat-bottomed glass tubes (BioTech Solutions); molded polytetrafluoroethylene caps (BioTech Solutions), polytetrafluoroethylene-coated stirring bars (BioTech Solutions), Eppendorf ThermoMixer, and 96-well deep-well plates.

2. Preparation of 0.01 M, pH 7.4 sodium phosphate buffer

15.6 g of $NaH_2PO_4 \cdot 2H_2O$ was weighed out, placed into a 1 L glass flask, and dissolved in 1 L of deionized water. The pH of the solution was approximately 4.7, and then the pH was adjusted to 7.4 with 10 M NaOH.

3. Solubility determination

1 mg of each compound powder was accurately weighed out and placed in a glass tube. The pH 7.4 sodium phosphate buffer was added to the glass tube, with 1 mL of the buffer added for each milligram of the compound powder. A stirring bar was added to each glass tube, and the glass tube was closed with a cap. The sample plate containing the glass tubes was placed into Eppendorf ThermoMixer and incubated at 25 °C at 1100 rpm for 24 h. After the incubation, the caps were removed, and the stirring bars were removed using a magnet. The observations in each glass tube were recorded. The plate was centrifuged at 25 °C at 4000 rpm for 30 min. 750 μL of supernatant was collected using a pipette. The tip was washed with acetonitrile for 5 s and then with purified water for 5 s. Then the first 50 μL was discarded as waste, and the remaining 700 μL was added to a 96-well sample plate containing glass tubes. The plate was then centrifuged for 30 min (25 °C, 4000 rpm). 10 μL of the sample of the second centrifugation was transferred using a pipette to 990 μL of a mixture of acetonitrile and water (1:1) containing an internal standard (100× sample). 10 μL of the diluted solution was transferred using a pipette to 990 μL of a mixture of acetonitrile and water (1:1) containing an internal standard (10,000× sample). The sample dilution factor may vary depending on the solubility level and the LC/MS signal response.

Table 20. The recorded observations and dilution factors

| Record | Observations after samples were left in phosphate buffer for 24 h | Dilution factor |
|---|---|---|
| No sign | A completely clear solution | 10000 |
| S | A clear solution with a small amount of solids | 10000 |
| S100 | A clear solution with a large amount of solids | 100 |
| 1 | A solution with a small amount of floating powder | 10000 |
| 2 | A suspension with a small amount of foam | 10000 |
| 3 | A solution with a large amount of floating powder | 100 |
| 4 | A suspension with a large amount of foam | 100 |

4. Preparation of standard

1 mg of each compound powder was accurately weighed out and added to a glass tube, and DMSO was added to each glass tube,
with 1 mL of DMSO added for each milligram of the compound powder. A stirring bar was added to each glass tube, and the glass tube was closed with a cap. The plate containing glass tubes of the standard was placed into Eppendorf ThermoMixer and incubated at 25 °C at 1,100 rpm for 2 h to fully dissolve the powders. Observations were made to see whether the solid had completely dissolved, and compounds that did not completely dissolve in the DMSO solution were recorded. 10 μL of 1 mg/mL standard was transferred using a pipette to 990 μL of a mixture of acetonitrile and water (1:1) containing an internal standard to give 10 μg/mL standard. 10 μL of 10 μg/mL standard was transferred using a pipette to 990 μL of a mixture of acetonitrile and water (1:1) containing an internal standard to give 0.1 μg/mL standard. The sample dilution factor may vary depending on the LC/MS signal response. Samples were analyzed by LC/MS/MS. All compounds were tested individually.

5. Calculations

All calculations were performed using Microsoft Excel. Samples were analyzed by LC/MS/MS and quantified based on the standard of a known concentration. The solubility of the test compounds was calculated using the following formula: [Sample] = Area Ratio sample × DF sample × [STD] / Area Ratio STD
DF: dilution factor.

Table 21. The solubility of some of the compounds of the present disclosure

| Example No. | Solubility (μM) |
|---|---|
| Compound A | 16 |
| Isomer 2 with a retention time of 1.887 min | 2126 |
| Isomer 1 with a retention time of 2.078 min | 2203 |

Test Example 5. PXR Induction Assay

1. The potential of the test compounds to induce the activity of drug metabolism enzymes through *in vitro* activation of PXR was evaluated. EC50 values for the test compounds were obtained by assessing the *in vitro* activation of PXR by different concentrations of the test compounds (30, 10, 3.33, 1.11, 0.370, and 0.123 μM). The concentrations of the positive control rifampicin were 20, 5, 1.25, 0.312, 0.0781, and 0.195 μM.
2. Materials and reagents

   1) DPX2 (HepG2 cells were stably transfected with the human PXR gene and the fluorescent reporter gene) cells were purchased from Puracyp (Carlsbad, CA).
   2) The test compounds were provided by the sponsor; the control drug (rifampicin) was purchased from Sigma (St. Louis, MO).
   3) The CellTiter-Fluor™ cell viability assay kit and the One-Glo fluorescence assay kit were purchased from Promega (Madison, WI); fetal bovine serum (FBS) was purchased from Corning (Manassas, VA); the MTS3 shaker was purchased from IKA Labortechnik (Staufen, Germany); DMEM, penicillin, and streptomycin were purchased from local suppliers; hygromycin B and G418 were purchased from Merck (Darmstadt, Germany); the cell culture medium and DPX2 cells were purchased from Puracyp Inc.

3. Experimental procedures

   3.1. Preparation for plating

      1) 50 mL of FBS was added to 450 mL of cell culture medium.
      2) DPX2 cells were cultured in a T-75 culture flask in a 37 °C, 5% CO2 incubator with 95% relative humidity. When covering 80-90% of the bottom of the culture flask, the cells were digested.
      3) The top layer of cells cultured in the T-75 culture flask was washed with 8 mL of PBS, and the PBS was pipetted off. The cells were digested with 3 mL of pancreatin at 37 °C for about 5 min or until they were floating in the pancreatin. 10 mL (excessive) of a serum-containing culture medium was added to neutralize the pancreatin.
      4) The cell suspension was transferred to a conical-bottomed centrifuge tube and centrifuged at 120 g for 10 min. The cells were suspended in the plating culture medium, and the concentration was adjusted to $4 \times 10^5$ cells/mL. The diluted cells were added to a 96-well cell culture plate at 100 μL/well. The culture plate was placed in an incubator and incubated at 37 °C for 24 h before the PXR activation experiment.

   3.2. Addition of compounds

      1) The test compounds and the positive compound (rifampicin) were prepared using DMSO, and the compounds were diluted with a serum-free culture medium at 37 °C. The final concentrations of the positive control rifampicin were 20, 5, 1.25, 0.312, 0.0781, and 0.195 μM. The final concentrations of the test compounds were 30, 10, 3.33, 1.11, 0.370, and 0.123 μM. The final concentration of DMSO was 0.1%. 1 μL of DMSO was added to a well containing 1 mL of pre-incubated culture medium as a solvent control.
      2) The cell culture plate was taken out of the incubator, and the culture medium was discarded. The test compounds and the positive compound were added to appropriate wells at 100 μL/well, and each group was tested in duplicate. The cell plate was placed in an incubator and incubated for 24 h.

   3.3. Quantification of PXR activation

      1) After 2 days of compound treatment, PXR activation was quantified for the culture.

2) The CellTiter-Fluor™ cell viability assay kit and the One-Glo Luciferase reagent were equilibrated to room temperature. The GF-AFC (10 μL) substrate was added to Assay Buffer (10 mL) to form a 2× reagent, and the reagent was then diluted to 1× with 10 mL of PBS; the ONE-Glo substrate was added to the ONE-Glo Luciferase Assay buffer.

3) The cell culture plate was taken out of the incubator, and the culture medium in each well was discarded. The plate was washed twice with PBS. The 1× CellTiter-Fluor™ reagent was added to a sterilized reagent reservoir, and 50 μL was transferred to each well using a multi-channel pipette. The plate was incubated at 37 °C for 30 min.

4) The 96-well cell plate was taken out of the incubator, and the fluorescence in each well was measured using a microplate reader put in the fluorescence mode at an excitation wavelength of 400 nm and an emission wavelength of 505 nm.

5) The ONE-Glo reagent was poured into a reagent reservoir, and then 50 μL was transferred to each well using a multi-channel pipette. The reagent was gently and well mixed, and the mixture was incubated and well mixed at room temperature for 5 min. After the incubation, the luminescence in each well was measured using a luminometer.

4. Calculation of cell induction values

4.1. Cell viability

The calculation formula for cell viability:

$$\text{Percent cell viability (\%)} = \text{I(sample)} / (\text{I(vehicle)} \times 100$$

I(sample) represents the fluorescence intensity of the sample, and I(vehicle) represents the fluorescence intensity of 0.1% DMSO to cells.

4.2. Calculation of cell induction values

All data were calculated using Microsoft Excel.

The luciferase activity was expressed in terms of RFU/RLU, where RLU is the average luminescence intensity value of two replicates of each concentration of each compound, and RFU is the average fluorescence intensity of two replicates of each concentration of each compound.

The calculation formula for fold induction:

$$\text{Fold induction} = \frac{RLU_{test}/RFU_{test}}{RLU_{vehicle}/RFU_{vehicle}}$$

Table 22. Some of the determined PXR induction values

| Compound | Compound concentration (μM) | Cell viability (%) | Fold induction |
|---|---|---|---|
| Rifampicin | 20.0 | 93.07 | 17.60 |
| | 0.0195 | 103.39 | 1.33 |
| Compound A | 10.0 | 94.32 | 48.51 |
| | 0.123 | 99.67 | 1.04 |
| Isomer 2 with a retention time of 1.887 min | 10.00 | 108.37 | 1.48 |
| | 0.123 | 102.41 | 0.99 |
| Isomer 1 with a retention time of 2.078 min | 10.00 | 117.18 | 1.43 |
| | 0.123 | 99.16 | 0.87 |

## Claims

1. A crystal form A of the compound (3S,4R)-4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)tetrahydro-2H-pyran-3-ol, wherein an X-ray powder diffraction pattern, expressed in terms of 2θ angles, which are diffraction angles, of the crystal form has characteristic peaks at 9.6, 10.0, 11.7, 15.0, 21.1, and 21.7, preferably at 7.8, 9.6, 10.0, 11.7, 15.0, 18.9, 19.4, 20.1, 21.1, and 21.7, and most preferably at 7.8, 9.6, 10.0, 11.7, 15.0, 18.9, 19.4, 20.1, 21.1, 21.7, 23.5, 26.7, and 29.4.

2. A crystal form B of the compound (3S,4R)-4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)tetrahydro-2H-pyran-3-ol, wherein an X-ray powder diffraction pattern, expressed in terms of 2θ angles, which are diffraction angles, of the crystal form has characteristic peaks at 11.3, 15.1, 20.9, 22.8, and 23.7, preferably at 7.4, 11.3, 14.3, 15.1, 16.3, 18.0, 20.0, 20.9, 22.8, 23.2, 23.7, 25.1, and 26.0, and most preferably at 7.4, 9.5, 11.3, 12.4, 12.8, 13.7, 14.3, 15.1, 16.3, 18.0, 19.2, 20.0, 20.9, 22.8, 23.2, 23.7, 25.1, 26.0, 26.8, 28.2, 30.4, 32.7, 33.6, 34.3, 35.5, 38.4, 39.0, and 40.5.

3. A crystal form C of the compound (3S,4R)-4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)tetrahydro-2H-pyran-3-ol, wherein an X-ray powder diffraction pattern, expressed in terms of 2θ angles, which are diffraction angles, of the crystal form has characteristic peaks at 4.8, 10.1, 12.0, and 15.0, preferably at 4.8, 7.6, 10.1, 12.0, 15.0, 19.7, 21.2, and 23.5.

4. A crystal form D of the compound (3S,4R)-4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)tetrahydro-2H-pyran-3-ol, wherein an X-ray powder diffraction pattern, expressed in terms of 2θ angles, which are diffraction angles, of the crystal form has characteristic peaks at 10.0, 10.5, 17.0, 18.7, and 23.9, preferably at 10.0, 10.5, 11.0, 13.4, 15.9, 17.0, 18.3, 18.7, 20.8, 23.9, and 28.0, and most preferably at 10.0, 10.5, 11.0, 12.5, 13.4, 15.9, 17.0, 18.3, 18.7, 20.1, 20.8, 22.3, 23.9, 26.7, 28.0, 30.5, 31.0, 32.1, 33.1, 33.8, and 34.6.

5. A crystal form E of the compound (3S,4R)-4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)tetrahydro-2H-pyran-3-ol, wherein an X-ray powder diffraction pattern, expressed in terms of 2θ angles, which are diffraction angles, of the crystal form has characteristic peaks at 4.8, 11.4, 14.4, 15.1, 16.4, 19.3, and 22.9, preferably at 4.8, 7.4, 11.4, 14.4, 15.1, 16.4, 17.9, 19.3, 19.9, 20.9, 22.9, and 23.5, and more preferably at 4.8, 7.4, 9.6, 11.4, 14.4, 15.1, 16.4, 17.9, 19.3, 19.9, 20.9, 22.9, 23.5, 24.2, 25.2, 25.9, 26.8, and 30.5.

6. A crystal form F of the compound (3S,4R)-4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)tetrahydro-2H-pyran-3-ol, wherein an X-ray powder diffraction pattern, expressed in terms of 2θ angles, which are diffraction angles, of the crystal form has characteristic peaks at 11.1, 11.4, 14.3, 15.1, 15.7, 19.2, and 22.0, preferably at 9.1, 11.1, 11.4, 13.3, 14.3, 15.1, 15.7, 16.6, 17.9, 19.2, 20.0, 21.1, 22.0, 23.6, 24.6, and 26.1, and most preferably at 4.8, 9.1, 9.8, 11.1, 11.4, 12.0, 13.3, 14.3, 15.1, 15.7, 16.6, 17.9, 19.2, 20.0, 21.1, 22.0, 22.5, 23.6, 24.2, 24.6, 25.2, 25.6, 26.1, 28.7, 30.3, and 35.0.

7. A crystal form G of the compound (3S,4R)-4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)tetrahydro-2H-pyran-3-ol, wherein an X-ray powder diffraction pattern, expressed in terms of 2θ angles, which are diffraction angles, of the crystal form has characteristic peaks at 11.3, 15.3, 16.1, 17.9, 18.6, and 20.8, preferably at 11.3, 15.3, 16.1, 17.9, 18.6, 20.8, 22.8, 23.1, 23.8, and 25.8, and most preferably at 9.1, 11.3, 12.6, 13.8, 14.7, 15.3, 16.1, 17.0, 17.5, 17.9, 18.6, 19.6, 20.8, 21.6, 22.8, 23.1, 23.8, 24.3, 25.3, 25.8, 27.3, 27.8, 29.3, 30.8, 32.3, 33.6, 34.5, and 35.0.

8. A crystal form H of the compound (3S,4R)-4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)tetrahydro-2H-pyran-3-ol, wherein an X-ray powder diffraction pattern, expressed in terms of 2θ angles, which are diffraction angles, of the crystal form has characteristic peaks at 11.7, 12.3, 15.5, 19.4, and 22.3, preferably at 11.7, 12.3, 15.5, 18.2, 19.4, 21.3, 22.3, 24.9, 27.1, and 28.1, and most preferably at 10.1, 11.7, 12.3, 12.8, 13.4, 15.5, 17.1, 18.2, 19.4, 20.5, 21.3, 22.3, 23.2, 24.5, 24.9, 25.6, 26.4, 27.1, 28.1, 28.4, 30.7, 31.2, 32.1, 33.3, 36.0, 37.0, and 39.2.

9. The crystal form according to any one of claims 1 to 8, wherein the 2θ values have a margin of error of ±0.2.

10. A method for preparing the crystal form A of the compound (3S,4R)-4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)tetrahydro-2H-pyran-3-ol according to claim 1 or 9, comprising the following steps:

method 1

a) dissolving the compound represented by formula (I) in a solvent 1, and
b) adding a solvent 2 and crystallizing,
wherein the solvent 1 is selected from the group consisting of a ketone solvent and an ester solvent, and the solvent 2 is an ether solvent; preferably,
the ketone solvent is selected from the group consisting of acetone and methyl isobutyl ketone, the ester solvent is ethyl acetate, and the ether solvent is selected from the group consisting of isopropyl ether and methyl tert-butyl ether; and

method 2
dissolving the compound represented by formula (I) in a solvent 3 and stirring for crystallization, wherein the solvent 3 is selected from the group consisting of an ester solvent and a ketone solvent; preferably, the ester solvent is selected from the group consisting of ethyl acetate and isopropyl acetate, and the ketone solvent is methyl isobutyl ketone.

**11.** A method for preparing the crystal form B of the compound (3S,4R)-4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)tetrahydro-2H-pyran-3-ol according to claim 2 or 9, comprising the following steps: dissolving the compound represented by formula (I) in a substituted lower alkane and stirring for crystallization, wherein preferably, the substituted lower alkane is nitromethane.

**12.** A method for preparing the crystal form C of the compound (3S,4R)-4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)tetrahydro-2H-pyran-3-ol according to claim 3 or 9, wherein the method is selected from the group consisting of the following methods:

method 1

dissolving the compound represented by formula (I) in a solvent 4 and volatilizing for crystallization, wherein the solvent 4 is selected from the group consisting of a substituted lower alkane and a ketone solvent; preferably, the substituted lower alkane is selected from the group consisting of dichloromethane and 1,2-dichloroethane, and the ketone solvent is methyl isobutyl ketone;

method 2
dissolving the compound represented by formula (I) in a solvent 5 and stirring for crystallization, wherein the solvent 5 is selected from the group consisting of an aromatic hydrocarbon solvent; preferably, the aromatic hydrocarbon solvent is selected from the group consisting of p-xylene and toluene; and
method 3

a) dissolving the compound represented by formula (I) in a solvent 6, and
b) adding a solvent 7 and crystallizing,

wherein the solvent 6 is selected from the group consisting of an alcohol solvent, a ketone solvent, a nitrile solvent, and an ether solvent, and the solvent 7 is an ether solvent; preferably, the alcohol solvent is selected from the group consisting of isopropanol and ethanol, the ketone solvent is acetone, the nitrile solvent is acetonitrile, and the ether solvent is selected from the group consisting of tetrahydrofuran and isopropyl ether.

**13.** A method for preparing the crystal form D of the compound (3S,4R)-4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)tetrahydro-2H-pyran-3-ol according to claim 4 or 9, comprising the following steps: dissolving the compound represented by formula (I) in a solvent 8 and stirring for crystallization, wherein the solvent 8 is an ether solvent or a mixed solvent of an alcohol solvent and an ether solvent; preferably, the alcohol solvent is methanol, ethanol, or isopropanol, and the ether solvent is isopropyl ether.

**14.** A method for preparing the crystal form E of the compound (3 S,4R)-4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)tetrahydro-2H-pyran-3-ol according to claim 5 or 9, wherein the method is selected from the group consisting of the following methods:

method 1
dissolving the compound represented by formula (I) in a solvent 9 and stirring for crystallization, wherein the solvent 9 is a nitrile solvent; preferably, the nitrile solvent is acetonitrile; and
method 2

a) dissolving the compound represented by formula (I) in a solvent 10, and
b) adding a solvent 11 and crystallizing,

wherein the solvent 10 is selected from the group consisting of a ketone solvent, and the solvent 11 is an ether solvent; preferably, the ketone solvent is acetone, and the ether solvent is isopropyl ether.

**15.** A method for preparing the crystal form F of the compound (3S,4R)-4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)tetrahydro-2H-pyran-3-ol according to claim 6 or 9, wherein the method is selected from the group consisting of the following methods:

method 1
dissolving the compound represented by formula (I) in a solvent 12 and stirring for crystallization, wherein the solvent 12 is a ketone solvent; preferably, the ketone solvent is methyl isobutyl ketone; and
method 2
dissolving the compound represented by formula (I) in tetrahydrofuran completely, adding isopropyl ether and a seed crystal of the crystal form F, and crystallizing.

**16.** A method for preparing the crystal form G of the compound (3S,4R)-4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)tetrahydro-2H-pyran-3-ol according to claim 7 or 9, comprising the following steps: dissolving the compound represented by formula (I) in a mixed solvent of an alcohol solvent and an ether solvent and stirring for crystallization, wherein preferably, the alcohol solvent is selected from the group consisting of methanol, and the ether solvent is isopropyl ether.

**17.** A method for preparing the crystal form H of the compound (3S,4R)-4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)tetrahydro-2H-pyran-3-ol according to claim 8 or 9, wherein the method is selected from the group consisting of the following methods:

method 1

dissolving the compound represented by formula (I) in a solvent 13 and stirring for crystallization, wherein the solvent 13 is selected from the group consisting of an alcohol solvent,
a mixed solvent of an alcohol solvent and water, a ketone solvent, and an ester solvent; preferably, the alcohol solvent is selected from the group consisting of methanol, ethanol, isopropanol, and n-propanol, the ketone solvent is selected from the group consisting of acetone, 2-butanone, and methyl isobutyl ketone, and the ester solvent is ethyl acetate;

method 2

mixing the compound represented by formula (I) with a solvent 14 and slurrying for crystallization, wherein the solvent 14 is selected from the group consisting of water and an ether solvent;
preferably, the ether solvent is selected from the group consisting of methyl tert-butyl ether and isopropyl ether; and

method 3

a) dissolving the compound represented by formula (I) in a solvent 15, and
b) adding a solvent 16 and crystallizing,

wherein the solvent 15 is selected from the group consisting of an alcohol solvent, a ketone solvent, and an ether solvent, and the solvent 16 is selected from the group consisting of a lower

alkane, an ether solvent, and water; preferably, the alcohol solvent is selected from the group consisting of isopropanol, the ketone solvent is acetone, the lower alkane is n-heptane, and the ether solvent is tetrahydrofuran, isopropyl ether, or methyl tert-butyl ether.

**18.** A pharmaceutically acceptable salt of the compound (3S,4R)-4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)tetrahydro-2H-pyran-3-ol, wherein the pharmaceutically acceptable salt is selected from the group consisting of a hydrochloride, a sulfate, a mesylate, a p-toluenesulfonate, a phosphate, a citrate, and a malate.

**19.** A method for preparing the pharmaceutically acceptable salt of the compound (3S,4R)-4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino) tetrahydro-2H-pyran-3-ol according to claim 18, comprising a step of reacting a free alkali with an acid molecule selected from the group consisting of hydrochloric acid, sulfuric acid, mesylic acid, p-toluenesulfonic acid, phosphoric acid, citric acid, and malic acid.

**20.** A pharmaceutical composition, comprising the crystal form or compound according to any one of claims 1-9 or claim 18 and optionally a pharmaceutically acceptable excipient.

**21.** A preparation method for a pharmaceutical composition, comprising a step of mixing the crystal form or compound according to any one of claims 1-9 or claim 18 with a pharmaceutically acceptable excipient.

**22.** Use of the crystal form or compound according to any one of claims 1-9 or claim 18 or the composition according to claim 20 in the preparation of a medicament for treating or preventing a cyclin-dependent kinase-associated disease.

**23.** Use of the crystal form or compound according to any one of claims 1-9 or claim 18 or the composition according to claim 20 in the preparation of a medicament for treating or preventing a cancer, wherein preferably, the cancer is selected from the group consisting of breast cancer, ovarian cancer, bladder cancer, uterine cancer, prostate cancer, lung cancer, esophageal cancer, head and neck cancer, intestinal cancer, renal cancer, liver cancer, pancreatic cancer, gastric cancer, and thyroid cancer.

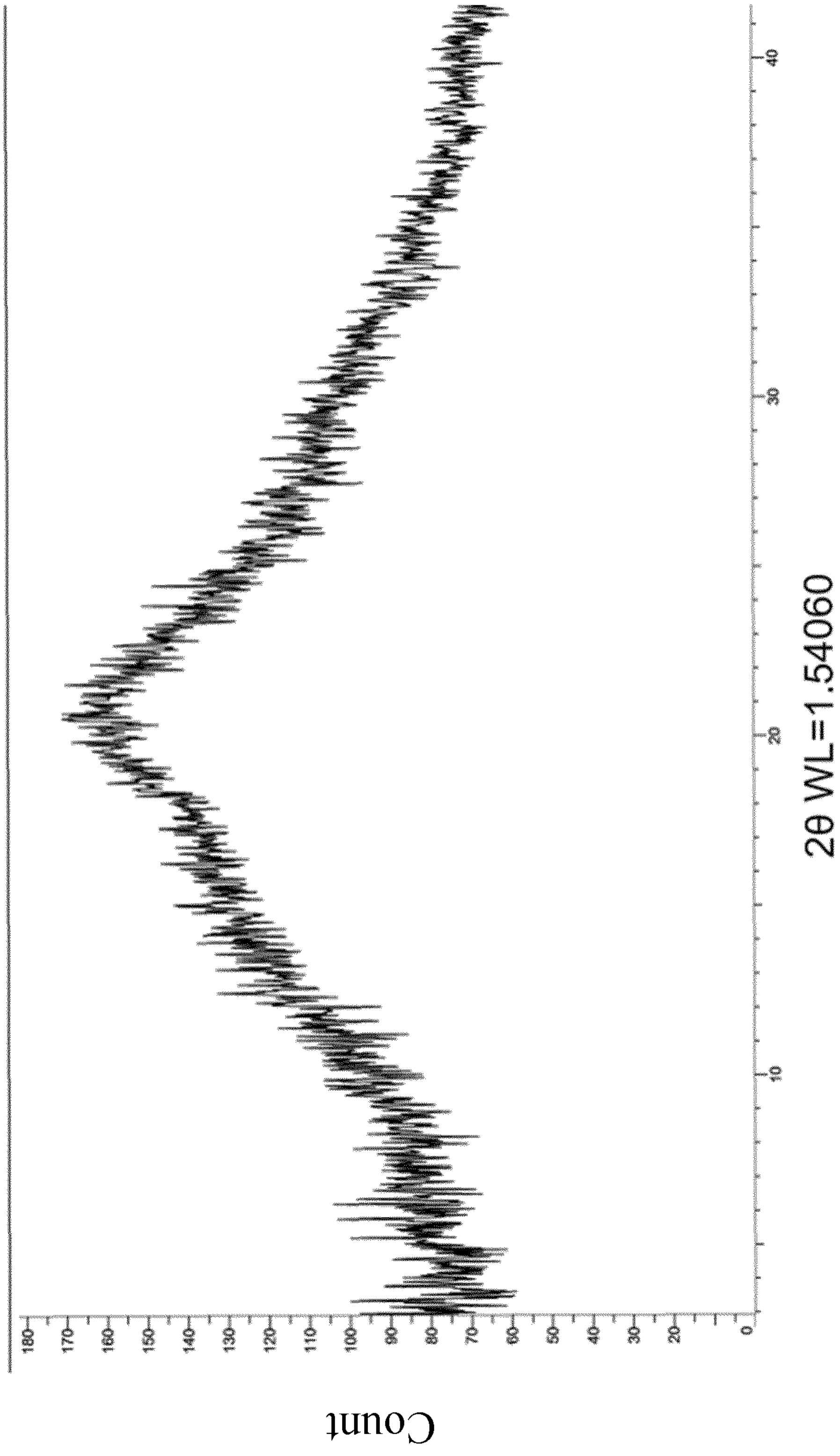


FIG. 1

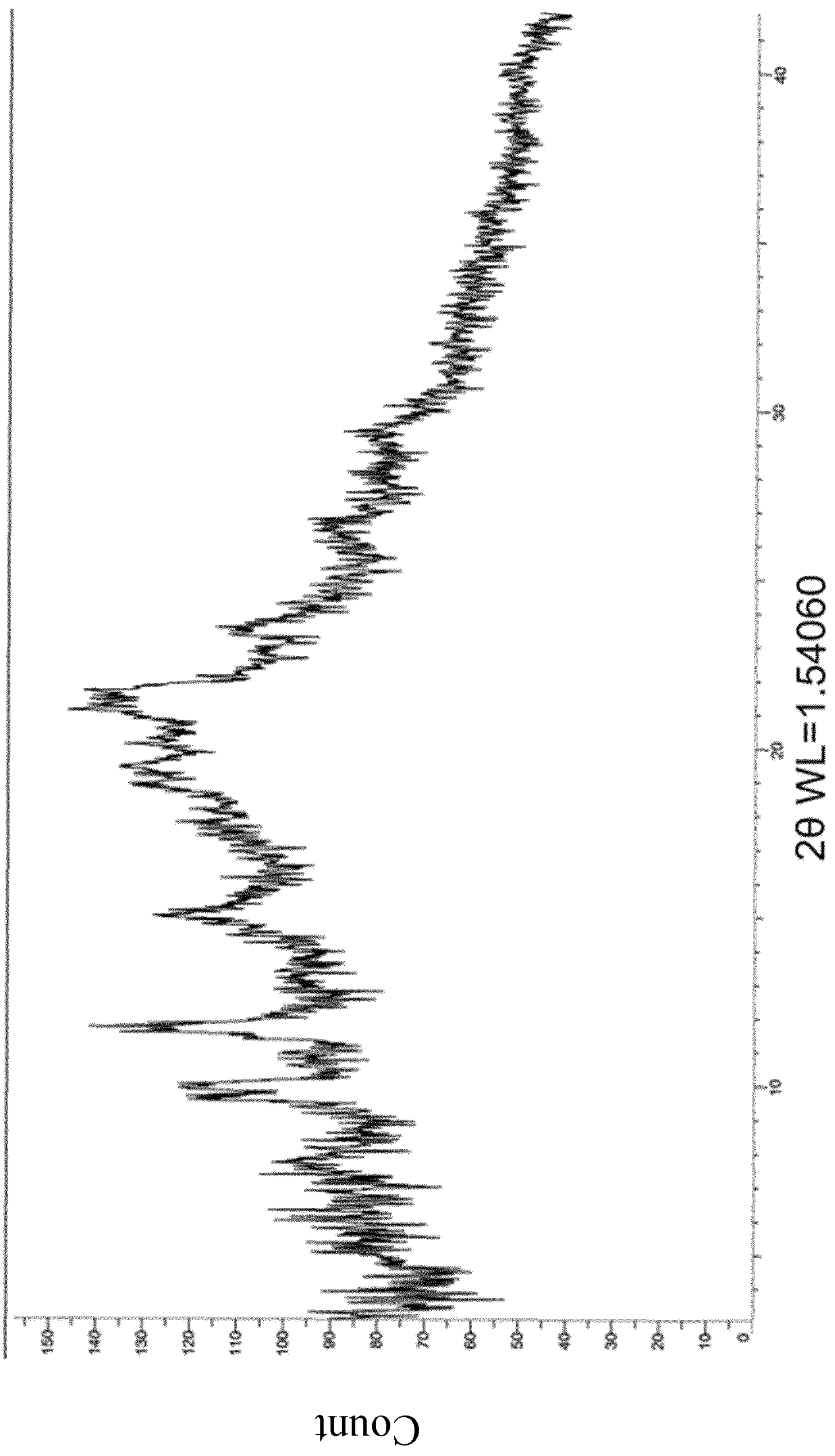
Count
0
10
20
30
40
50
60
70
80
90
100
110
120
130
140
150
10
20
30
40
2θ WL=1.54060

FIG. 2

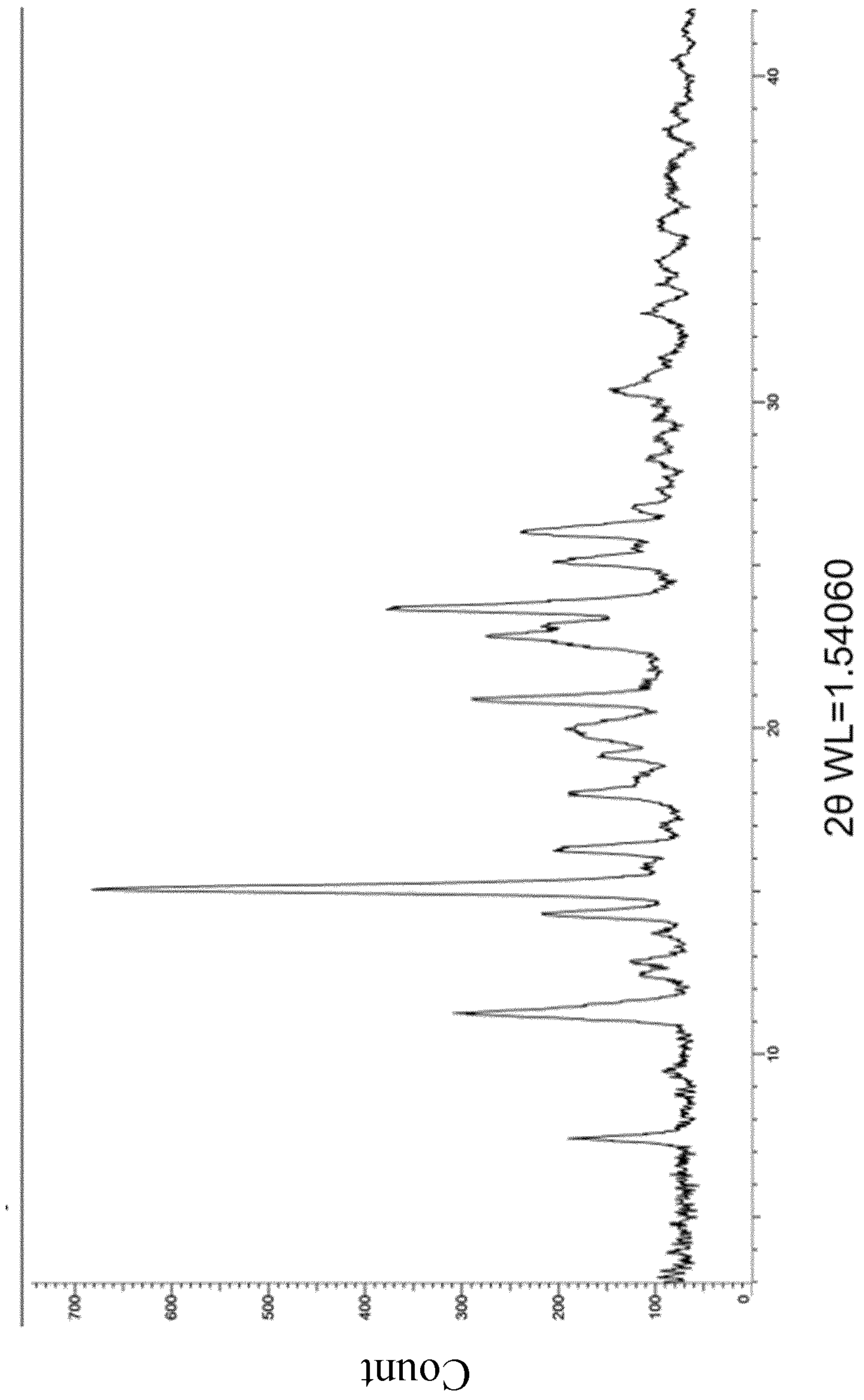


FIG. 3

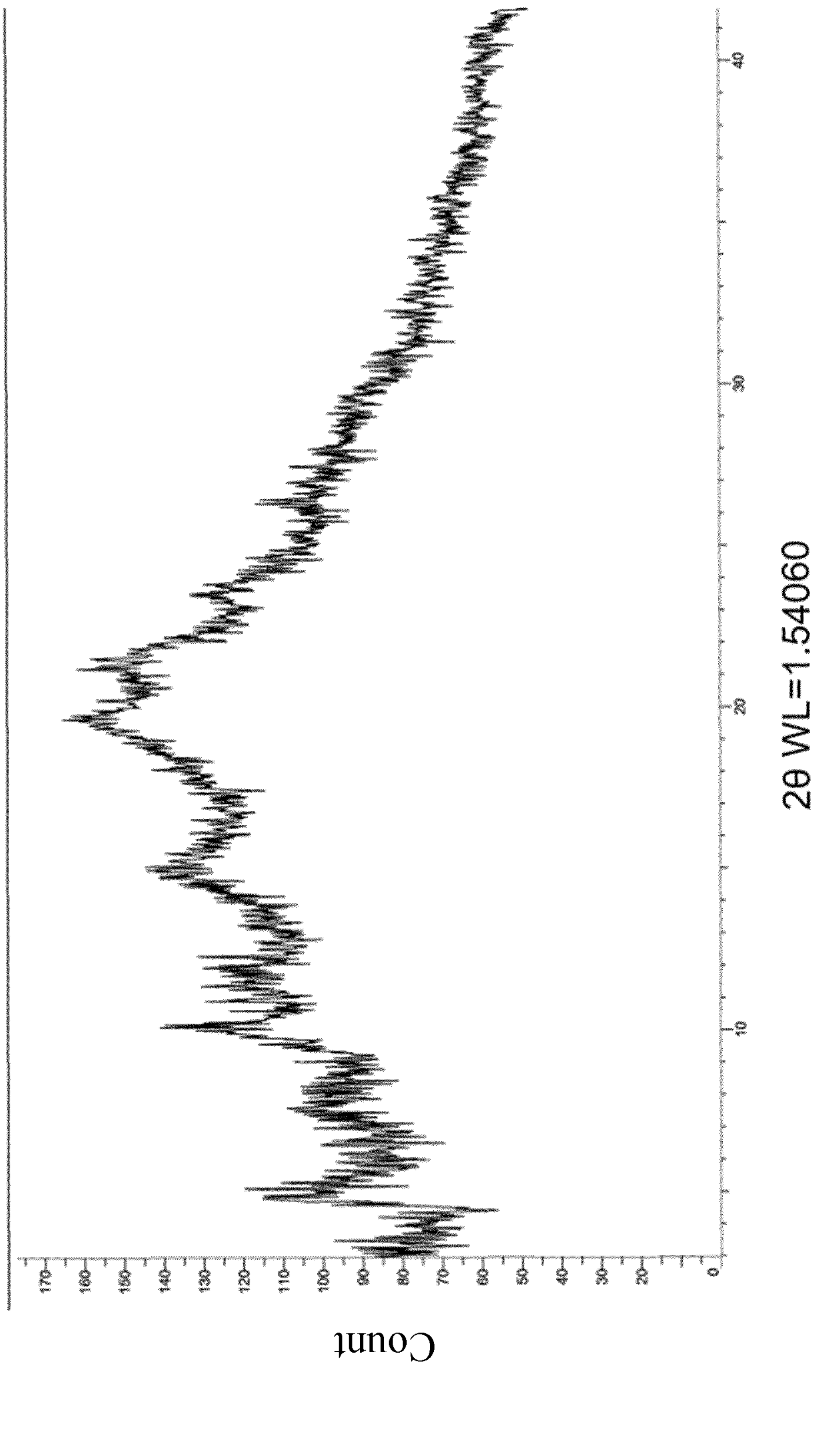
Count
170
160
150
140
130
120
110
100
90
80
70
60
50
40
30
20
0
10
20
30
40
2θ WL=1.54060

FIG. 4

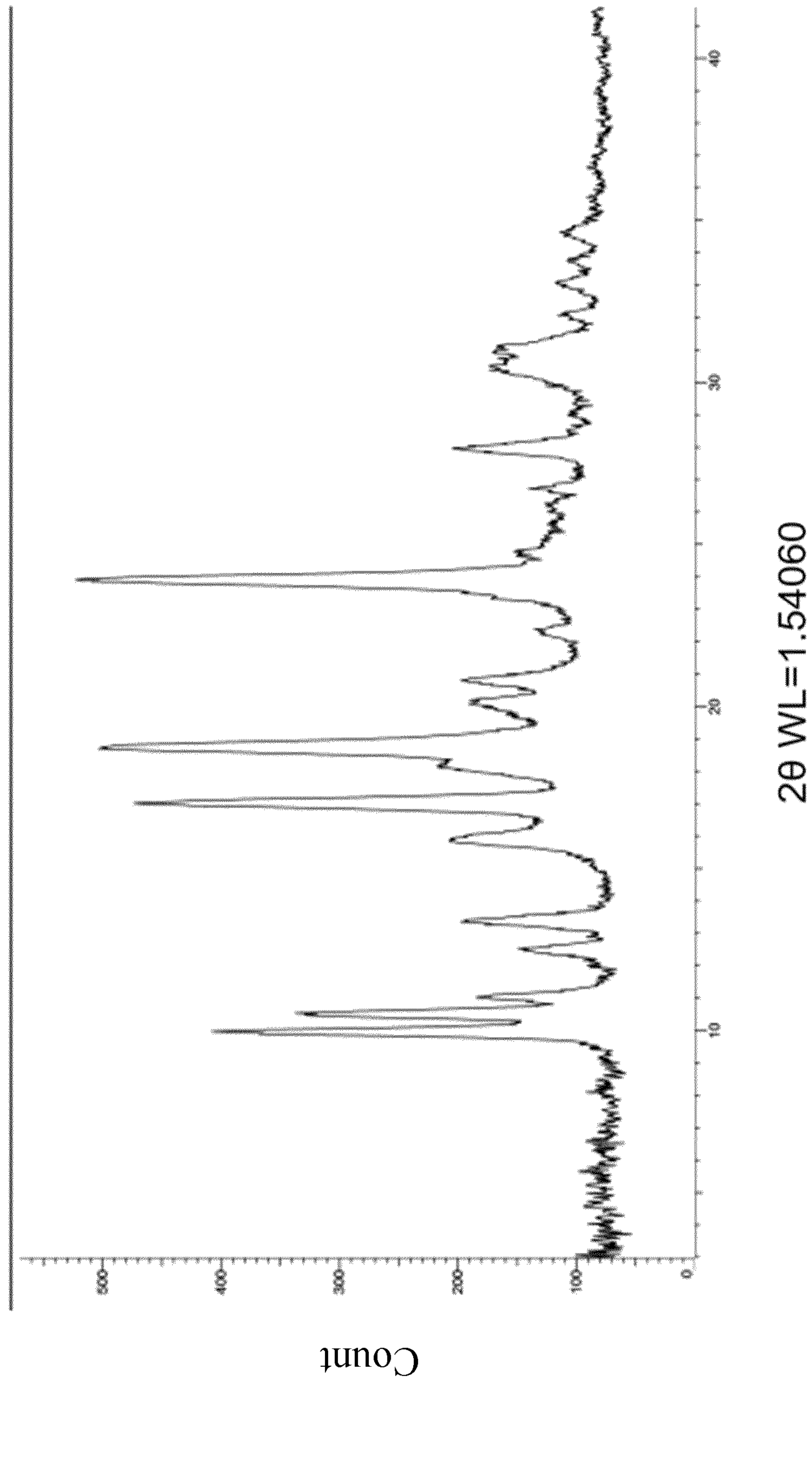


FIG. 5

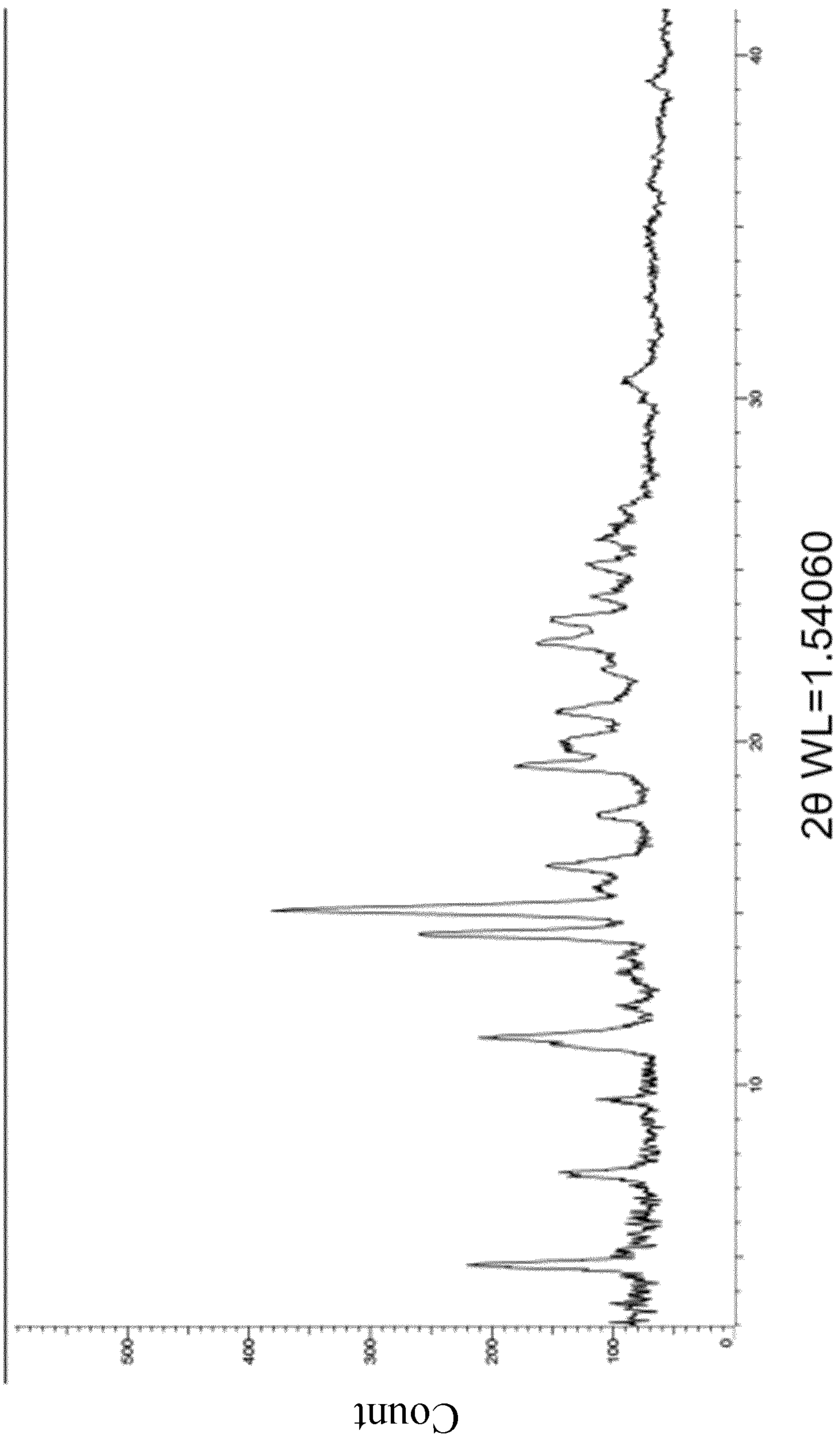


FIG. 6

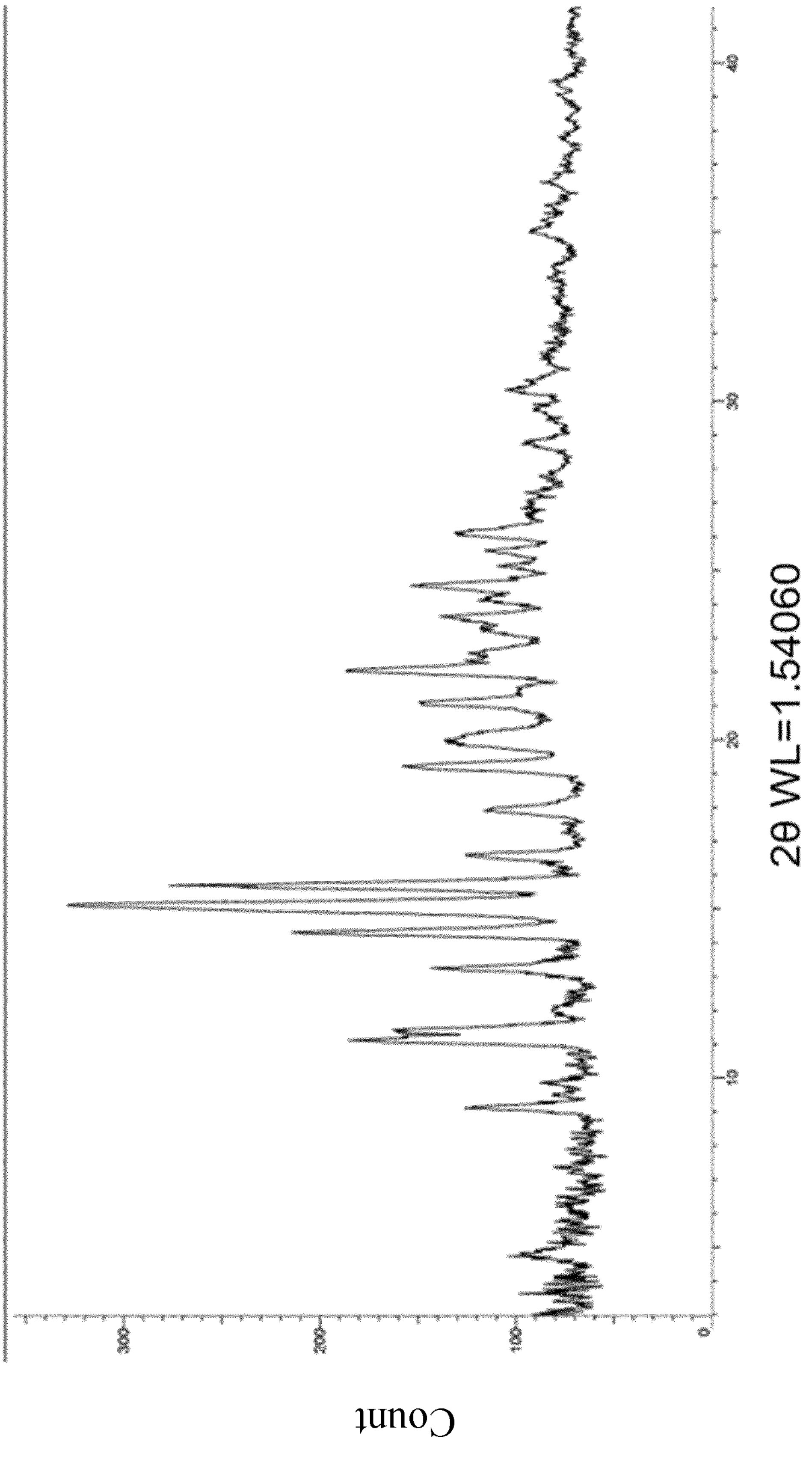


FIG. 7

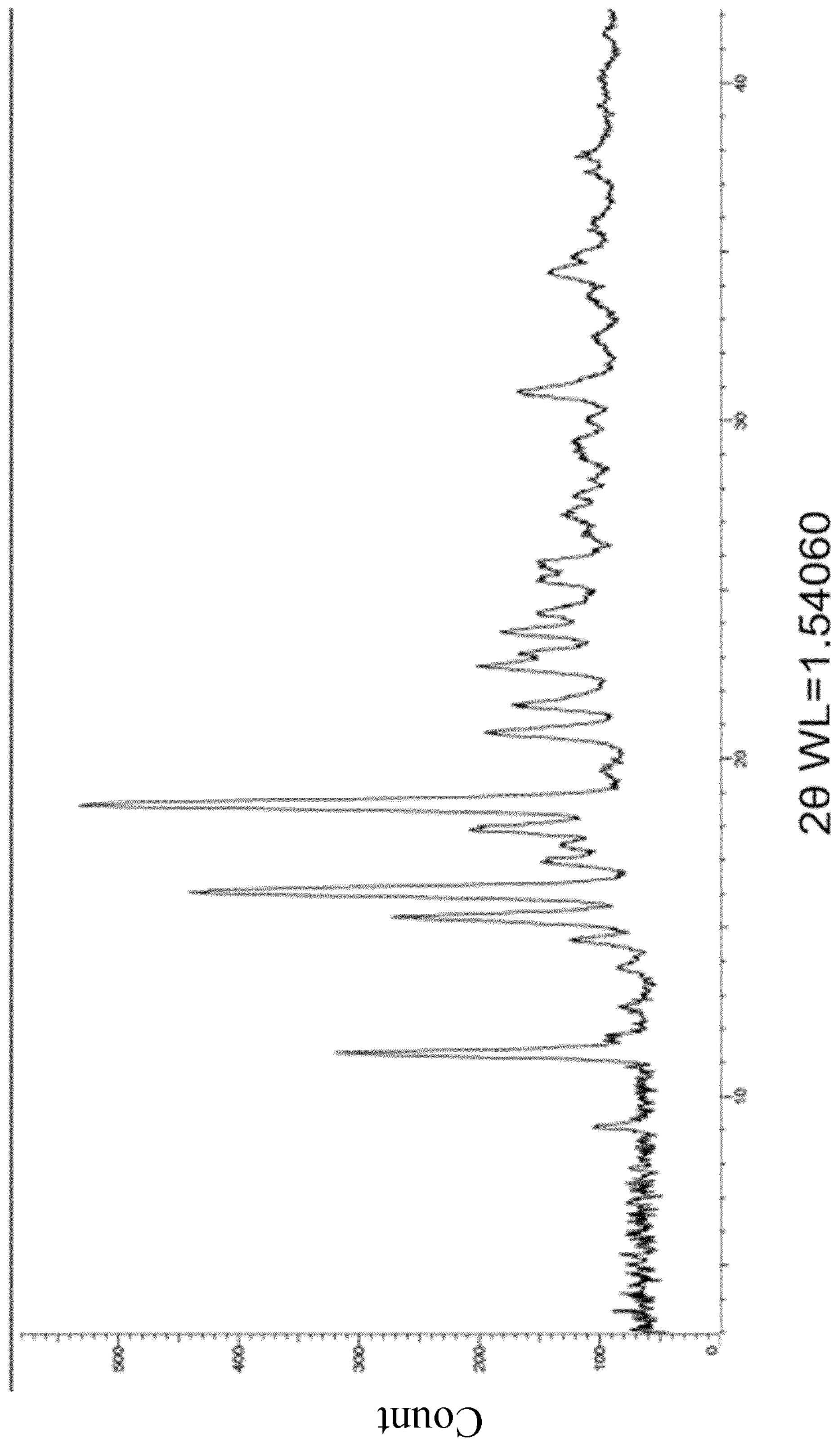


FIG. 8

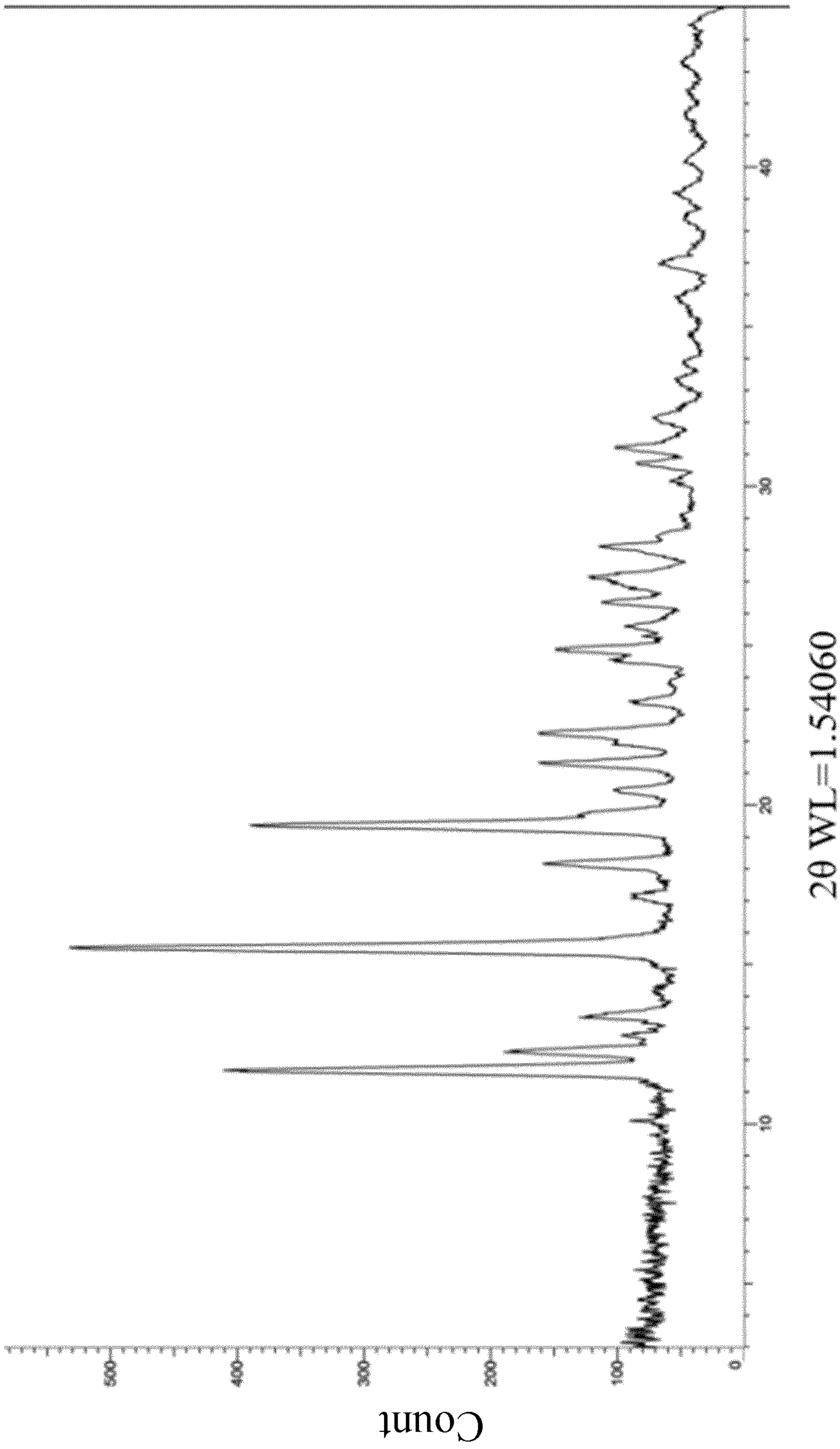


FIG. 9

**INTERNATIONAL SEARCH REPORT**

International application No.
**PCT/CN2023/110180**

**A. CLASSIFICATION OF SUBJECT MATTER**

C07D498/06(2006.01)i; A61K31/5383(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:C07D,A61K.A61P

Documentation searched other than minimum documentation to the extent that such documents are included in the fields searched

Electronic data base consulted during the international search (name of data base and, where practicable, search terms used)

CNTXT, WPABS, ENTXTC, CAPLUS (STN), REGISTRY (STN), CNKI: 恒瑞医药, 拓界生物, 癌, 肿瘤, 细胞周期蛋白, 依赖性激酶, 三环, 二氮杂苊烯, CDK?, cancer, tumor, tumour, tricycl+, diazaacenaphthen+, 结构式检索, structural formula search.

**C. DOCUMENTS CONSIDERED TO BE RELEVANT**

| Category* | Citation of document, with indication, where appropriate, of the relevant passages | Relevant to claim No. |
|---|---|---|
| PX | WO 2022166799 A1 (TUOJIE BIOTECH (SHANGHAI) CO., LTD.) 11 August 2022 (2022-08-11)<br>description, pages 26-28, embodiment 4 | 1-23 |
| A | CN 112313219 A (PFIZER INC.) 02 February 2021 (2021-02-02)<br>claims 1 and 21, and description, pages 144-145 | 1-23 |
| A | CN 113698391 A (TUOJIE BIOTECH (SHANGHAI) CO., LTD.) 26 November 2021 (2021-11-26)<br>Claims 1-18 and 25, and the third compound in claim 21. | 1-23 |
| A | CN 110234652 A (BETTA PHARMACEUTICALS CO., LTD.) 13 September 2019 (2019-09-13)<br>claims 1-34, and description, page 28 | 1-23 |
| A | CN 110818690 A (SHENZHEN TARGETRX, INC.) 21 February 2020 (2020-02-21)<br>claims 1-14, and description, paragraph [1007] | 1-23 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 October 2023** | **23 October 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2023/110180**

| Patent document cited in search report | Publication date (day/month/year) | Patent family member(s) | Publication date (day/month/year) |
|---|---|---|---|
| WO 2022166799 A1 | 11 August 2022 | CN 116583524 A | 11 August 2023 |
| | | CA 3206898 A1 | 11 August 2022 |
| | | AU 2022217309 A1 | 14 September 2023 |
| | | TW 202231282 A | 16 August 2022 |
| CN 112313219 A | 02 February 2021 | US 2019330196 A1 | 31 October 2019 |
| | | US 10766884 B2 | 08 September 2020 |
| | | US 2022089580 A1 | 24 March 2022 |
| | | EP 3784664 A1 | 03 March 2021 |
| | | ECSP 20067394 A | 31 December 2020 |
| | | CL 2020002748 A1 | 12 February 2021 |
| | | MX 2020011294 A | 18 November 2020 |
| | | DOP 2020000191 A | 28 February 2021 |
| | | RU 2021136543 A | 16 December 2021 |
| | | MA 52360 A | 03 March 2021 |
| | | TW 202000661 A | 01 January 2020 |
| | | TWI 740135 B | 21 September 2021 |
| | | CO 2020013149 A2 | 10 November 2020 |
| | | CU 20200070 A7 | 12 May 2021 |
| | | PE 20201202 A1 | 11 November 2020 |
| | | US 2020354350 A1 | 12 November 2020 |
| | | US 11220494 B2 | 11 January 2022 |
| | | CR 20200503 A | 17 December 2020 |
| | | GEP 20227433 B | 25 October 2022 |
| | | BR 112020021689 A2 | 26 January 2021 |
| | | BR 112020021689 B1 | 28 February 2023 |
| | | UA 126177 C2 | 25 August 2022 |
| | | JP 2021522275 A | 30 August 2021 |
| | | JP 7089061 B2 | 21 June 2022 |
| | | CA 3098283 A1 | 31 October 2019 |
| | | CA 3098283 C | 23 May 2023 |
| | | KR 20210002642 A | 08 January 2021 |
| | | ZA 202006944 B | 26 July 2023 |
| | | RU 2762557 C1 | 21 December 2021 |
| | | NI 202000072 A | 23 March 2021 |
| | | JP 2022120096 A | 17 August 2022 |
| | | WO 2019207463 A1 | 31 October 2019 |
| | | UY 38196 A | 29 November 2019 |
| | | IL 278075 A | 30 November 2020 |
| | | IL 278075 B1 | 01 January 2023 |
| | | IL 278075 B2 | 01 May 2023 |
| | | AU 2019259653 A1 | 29 October 2020 |
| | | AU 2019259653 B2 | 19 January 2023 |
| | | PH 12020551692 A1 | 19 July 2021 |
| | | SG 11202009992 VA | 27 November 2020 |
| CN 113698391 A | 26 November 2021 | None | |
| CN 110234652 A | 13 September 2019 | KR 20190114971 A | 10 October 2019 |
| | | KR 102543603 B1 | 14 June 2023 |
| | | EP 3559000 A1 | 30 October 2019 |
| | | EP 3559000 A4 | 27 May 2020 |
| | | EP 3559000 B1 | 23 March 2022 |

Form PCT/ISA/210 (patent family annex) (July 2022)

**INTERNATIONAL SEARCH REPORT**
**Information on patent family members**

International application No.
**PCT/CN2023/110180**

| Patent document cited in search report | Publication date (day/month/year) | Patent family member(s) | Publication date (day/month/year) |
|---|---|---|---|
| | | CA 3047876 A1 | 28 June 2018 |
| | | CA 3047876 C | 29 August 2023 |
| | | DK 3559000 T3 | 07 June 2022 |
| | | AU 2017379041 A1 | 25 July 2019 |
| | | AU 2017379041 B2 | 26 August 2021 |
| | | JP 2020504747 A | 13 February 2020 |
| | | JP 7105781 B2 | 25 July 2022 |
| | | TW 201829406 A | 16 August 2018 |
| | | TWI 732083 B | 01 July 2021 |
| | | WO 2018113771 A1 | 28 June 2018 |
| | | US 2021130345 A1 | 06 May 2021 |
| | | US 11053238 B2 | 06 July 2021 |
| | | ES 2917377 T3 | 08 July 2022 |
| | | RU 2019121937 A | 22 January 2021 |
| | | RU 2019121937 A3 | 09 February 2021 |
| CN 110818690 A | 21 February 2020 | CN 108602802 A | 28 September 2018 |
| | | US 2019152954 A1 | 23 May 2019 |
| | | WO 2018019204 A1 | 01 February 2018 |
| | | WO 2020151742 A1 | 30 July 2020 |
| | | EP 3492462 A1 | 05 June 2019 |
| | | JP 2019522011 A1 | 08 August 2019 |
| | | JP 2020183432 A1 | 12 November 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202210915848
- CN 2022074509 W
- WO 2019207463 A1

### Non-patent literature cited in the description

- *Annu. Rev. Pharmacol. Toxicol.*, 1999, vol. 39, 295-312
- *Annu. Rev. Cell. Dev. Biol.*, 1997, vol. 13, 261-291